# EUROPEAN PATENT SPECIFICATION

(11) **EP 1 298 138 B1**
(45) Date of publication and mention of the grant of the patent: **02.11.2006**
(21) Application number: 02028397.4
(22) Date of filing: 12.11.1999
(51) Int. Cl.: C07H 17/08, A61K 31/70, A61P 33/02

(54) **Carbamate and Carbazate Ketolide Antibiotics**
Carbamat und Carbazat Ketolid Antibiotika
Dérivés anitiobiotiques de Carbamate et de Carbazate Kètolides

(30) Priority: 10.12.1998 US 111728 P
(43) Date of publication of application: 02.04.2003
(62) Divisional of application: 99952753.4
(73) Proprietor: Pfizer Products Inc., Groton, Connecticut 06340 (US)
(72) Inventor: Kaneko, Takushi, Guilford, Connecticut 06437 (US); Su, Wei-Guo, East Lyme, Connecticut 06333 (US); Wu, Yong-Jin, East Lyme, Connecticut 06333 (US)
(74) Representative: Lane, Graham Mark Hamilton

(56) References cited:
- EP-A- 0 676 409
- WO-A-00/17218
- WO-A-98/56800
- WO-A-99/21866
- WO-A-99/35157
- FR-A- 2 732 023

## Description

### Background Of The Invention

This invention relates to novel macrolide compounds that are useful as antibacterial and antiprotozoal agents in mammals, including man, as well as in fish and birds. This invention also relates to pharmaceutical compositions containing the novel compounds and to methods of treating bacterial and protozoal infections in mammals, fish and birds by administering the novel compounds to mammals, fish and birds requiring such treatment.

Macrolide antibiotics are known to be useful in the treatment of a broad spectrum of bacterial and protozoal infections in mammals, fish and birds. Such antibiotics include various derivatives of erythromycin A such as azithromycin which is commercially available and is referred to in United States patents 4,474,768 and 4,517,359, both of which are incorporated herein by reference in their entirety. Other macrolide antibiotics are disclosed and claimed in PCT international application number PCT/IB98/00741, filed May 15, 1998 (Attorney docket number PC 9726A), which designates the United States, United States Patent 5,527,780, issued June 18, 1996, and U.S. provisional application number 60/101263 (filed September 22, 1998)(attorney docket number PC 10406). United States Patent 5,527,780, PCT international application number PCT/IB98/00741, and U.S. provisional application number 60/101263 are incorporated herein by reference in their entirety. Like azithromycin and other macrolide antibiotics, the novel macrolide compounds of the present invention possess activity against various bacterial and protozoal infections as described below.

### Summary of the Invention

The present invention relates to compounds of the formula 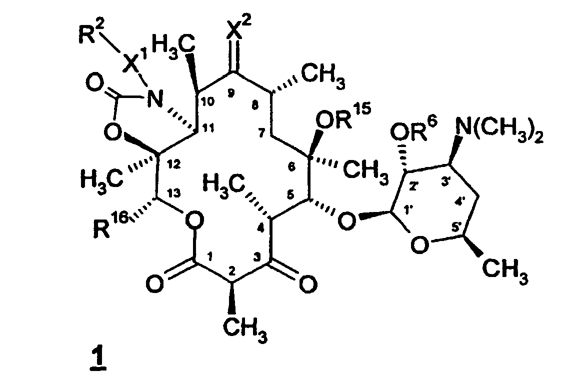 and to pharmaceutically acceptable salts and solvates thereof, wherein:
X¹ is -NR⁴-;
X² is =NOR¹;
R¹ is H, methyl or ethyl;
R² is -C(R⁷)(R⁸)C(R⁹)(R¹⁰)C(R¹¹)(R ¹²)(4-10 membered heterocyclic) or -(CR⁷R⁸)ᵣ(C₃-C₇ cycloalkylene)(CR⁹R¹⁰)_{q}(4-10 membered heterocyclic), wherein q and r are each integers independently ranging from 0 to 2, and the heterocyclic moiety of the foregoing groups is substituted by -(CR⁴R⁵)ₜ(4-10 membered heterocyclic) or -(CR⁴R⁵)ₜ(C₆-C₁₀ aryl) wherein t is an integer from 0 to 5, and the aryl and heterocyclic moieties of each of the foregoing groups are optionally substituted by 1 to 4 R³ groups;
each R³ is independently selected from the group consisting of halo, cyano, nitro, trifluoromethyl, trifluoromethoxy, azido, -C(O)R⁵, -C(O)OR⁵, -OC(O)R⁵, -NR⁵C(O)R⁴, -C(O)NR⁴R⁵, -NR⁴R⁵, hydroxy, C₁-C₆ alkyl, C₂-C₆ alkenyl, C₂-C₆ alkynyl, C₆-C₁₀ aryl, 4-10 membered heterocyclic, and C₁-C₆ alkoxy;
each R⁴ and R⁵ is independently selected from H and C₁-C₆ alkyl;
R⁶ is H or -C(O)(C₁-C₆ alkyl);
R⁷, R⁸, R⁹, R¹⁰, R¹¹, R¹², R¹³ and R¹⁴ are each independently selected from H, halo and C₁-C₆ alkyl, provided that where X¹ is -NR⁴- and R² is -C(R⁷)(R⁸)C(R⁹)(R¹⁰)C(R¹¹)(R¹²)(4-10 membered heterocyclic), at least one of R⁷, R⁸, R⁹, R¹⁰, R¹¹, and R¹² is halo or C₁-C₆ alkyl;
or R⁷ and R⁸ are taken together with the carbon to which each is attached to form a C₃-C₇ cycloalkyl group and R⁹, R¹⁰, R¹¹, R¹², R¹³ and R¹⁴ are each independently selected from H, halo and C₁-C₆ alkyl;
or R⁹ and R¹⁰ are taken together with the carbon to which each is attached to form a C₃-C₆ cycloalkyl group and R⁷, R⁸, R¹¹, R¹², R¹³ and R¹⁴ are each independently selected from H, halo and C₁-C₆ alkyl;
or R¹¹ and R¹² are taken together with the carbon to which each is attached to form a C₃-C₇ cycloalkyl group and R⁷, R⁸, R⁹, R¹⁰, R¹³ and R¹⁴ are each independently selected from H, halo and C₁-C₆ alkyl;
R¹⁵ is C₁-C₆ alkyl; and
R¹⁶ is H or C₁-C₁₀ alkyl.

Specific embodiments of the present invention include compounds of formula **1** wherein X² is =NOR¹, R¹⁵ is methyl, R¹⁶ is ethyl, R⁶ is H or acetyl, X¹ is -NR⁴- and R² is -C(R⁷)(R⁸)C(R⁹)(R¹⁰)C(R¹¹)(R¹²)(4-10 membered heterocyclic) wherein said heterocyclic group is substituted by 4-10 membered heterocyclic and the foregoing heterocyclic moieties are optionally substituted by 1 or 2 substituents independently selected from halo and C₁-C₃ alkyl. More specifically, X¹ is -NH- and R² is -C(R⁷)(R⁸)C(R⁹)(R¹⁰)C(R¹¹)(R¹²)(4-pyridin-3-yl-imidazol-1-yl), and
(a) R⁷, R⁸, R⁹ and R¹⁰ are each H, R¹¹ is methyl or ethyl, and R¹² is H, methyl or ethyl; or
(b) R⁷, R⁸, R¹¹ and R¹² are each H, R⁹ is methyl or ethyl, and R¹⁰ is H, methyl or ethyl; or
(c) R⁹, R¹⁰, R¹¹ and R¹² are each H, R⁷ is methyl or ethyl, and R⁸ is H, methyl or ethyl.

Specific embodiments of the present invention include the following compounds:
11-Deoxy-5-O-desosaminyl-11-(3,3-dimethyl-3-(4-pyridin-3-yl-imidazol-1-yl-propyl))hydrazo-6-O-methyl-3-oxoerythronolide A, 11,12-carbamate, 9-E-(O-methyl)oxime;
11-Deoxy-5-O-desosaminyl-11-(3(R)-methyl-3-(4-pyridin-3-yl-imidazol-1-yl-propyl))hydrazo-6-O-methyl-3-oxoerythronolide A, 11,12-carbamate, 9-E-(O-methyl)oxime;
11-Deoxy-5-O-desosaminyl-11-(3(R)-ethyll-3-(4-pyridin-3-yl-imidazol-1-yl-propyl))hydrazo-6-O-methyl-3-oxoerythronolide A, 11,12-carbamate, 9-E-(O-methyl)oxime;
11-Deoxy-5-O-desosaminyl-11-(3(S)-methyl-3-(4-pyridin-3-yl-imidazol-1-yl-propyl))hydrazo-6-O-methyl-3-oxoerythronolide A, 11,12-carbamate, 9-E-(O-methyl)oxime;
11-Deoxy-5-O-desosaminyl-11-(3(S)-ethyl-3-(4-pyridin-3-yl-imidazol-1-yl-propyl))hydrazo-6-O-methyl-3-oxoerythronolide A, 11,12-carbamate, 9-E-(O-methyl)oxime;
11-Deoxy-5-O-desosaminyl-11-(2-(1-(4-pyridin-3-yl-imidazol-1-yl)-cyclopropyl)-ethyl)hydrazo-6-O-methyl-3-oxoerythronolide A, 11,12-carbamate, 9-E-(O-methyl)oxime;
11-Deoxy-5-O-desosaminyl-11-(2-(1-(4-pyridin-3-yl-imidazol-1-yl)-cyclobutyl)-ethyl)hydrazo-6-O-methyl-3-oxoerythronolide A, 11,12-carbamate, 9-E-(O-methyl)oxime;
11-Deoxy-5-O-desosaminyl-11-(2,2-dimethyl-3-(4-pyridin-3-yl-imidazol-1-yl-propyl))hydrazo-6-O-methyl-3-oxoerythronolide A, 11,12-carbamate, 9-E-(O-methyl)oxime;
11-Deoxy-5-O-desosaminyl-11-(2,2-diethyl-3-(4-pyridin-3-yl-imidazol-1-yl-propyl))hydrazo-6-O-methyl-3-oxoerythronolide A, 11,12-carbamate, 9-E-(O-methyl)oxime;
11-Deoxy-5-O-desosaminyl-11-(2,2-difluro-3-(4-pyridin-3-yl-imidazol-1-yl-propyl))hydrazo-6-O-methyl-3-oxoerythronolide A, 11,12-carbamate, 9-E-(O-methyl)oxime;
11-Deoxy-5-O-desosaminyl-11-(2,2-dichloro-3-(4-pyridin-3-yl-imidazol-1-yl-propyl))hydrazo-6-O-methyl-3-oxoerythronolide A, 11,12-carbamate, 9-E-(O-methyl)oxime;
11-Deoxy-5-O-desosaminyl-11-(2,2-dibromo-3-(4-pyridin-3-yl-imidazol-1-yl-propyl))hydrazo-6-O-methyl-3-oxoerythronolide A, 11,12-carbamate, 9-E-(O-methyl)oxime;
11-Deoxy-5-O-desosaminyl-11-(1-(4-pyridin-3-yl-imidazol-1-yl-methyl)-cyclopropyl)-methyl)hydrazo-6-O-methyl-3-oxoerythronolide A, 11,12-carbamate, 9-E-(O-methyl)oxime;
11-Deoxy-5-O-desosaminyl-11-(1-(4-pyridin-3-yl-imidazol-1-yl-methyl)-cyclobutyl)-methyl)hydrazo-6-O-methyl-3-oxoerythronolide A, 11,12-carbamate, 9-E-(O-methyl)oxime;
11-Deoxy-5-O-desosaminyl-11-(2(R)-methyl-3-(4-pyridin-3-yl-imidazol-1-yl-propyl))hydrazo-6-O-methyl-3-oxoerythronolide A, 11,12-carbamate, 9-E-(O-methyl)oxime;
11-Deoxy-5-O-desosaminyl-11-(2(R)-ethyl-3-(4-pyridin-3-yl-imidazol-1-yl-propyl))hydrazo-6-O-methyl-3-oxoerythronolide A, 11,12-carbamate, 9-E-(O-methyl)oxime;
11-Deoxy-5-O-desosaminyl-11-(2(S)-methyl-3-(4-pyridin-3-yl-imidazol-1-yl-propyl))hydrazo-6-O-methyl-3-oxoerythronolide A, 11,12-carbamate, 9-E-(O-methyl)oxime;
11-Deoxy-5-O-desosaminyl-11-(2(S)-ethyl-3-(4-pyridin-3-yl-imidazol-1-yl-propyl))hydrazo-6-O-methyl-3-oxoerythronolide A, 11,12-carbamate, 9-E-(O-methyl)oxime;
11-Deoxy-5-O-desosaminyl-1-(2(R)-fluro-3-(4-pyridin-3-yl-imidazol-1-yl-propyl))hydrazo-6-O-methyl-3-oxoerythronolide A, 11,12-carbamate, 9-E-(O-methyl)oxime;
11-Deoxy-5-O-desosaminyl-11-(2(S)-flurol-3-(4-pyridin-3-yl-imidazol-1-yl-propyl))hydrazo-6-O-methyl-3-oxoerythronolide A, 11,12-carbamate, 9-E-(O-methyl)oxime;
and the pharmaceutically acceptable salts and solvates of the foregoing compounds.

The invention also relates to a pharmaceutical composition for the treatment of a bacterial infection or a protozoa infection, or a disorder related to a bacterial or protozoal infection, in a mammal, fish, or bird, which comprises a therapeutically effective amount of a compound of formula **1**, or a pharmaceutically acceptable salt or solvate thereof, and a pharmaceutically acceptable carrier.

The invention also relates to a method of treating a bacterial infection or a protozoa infection, or a disorder related to a bacterial or protozoal infection, in a mammal, fish, or bird which comprises administering to said mammal, fish or bird a therapeutically effective amount of a compound of formula **1** or a pharmaceutically acceptable salt or solvate thereof.

The term "treating", as used herein, unless otherwise indicated, means reversing, alleviating, inhibiting the progress of, or preventing the disorder or condition to which such term applies, or one or more symptoms of such disorder or condition. The term "treatment', as used herein, refers to the act of treating, as "treating" is defined immediately above.

As used herein, unless otherwise indicated, the terms or phrases "bacterial infection(s)", "protozoal infection(s)", and "disorders related to bacterial infections or protozoal infections" include the following: pneumonia, otitis media, sinusitus, bronchitis, tonsillitis, and mastoiditis related to infection by *Streptococcus pneumoniae, Haemophilus influenzae, Moraxella catarrhalis, Staphylococcus aureus, Enterococcus faecalis, E. faecium, E. casselflavus, S. epidermidis, S. haemolyticus,* or *Peptostreptococcus* spp.; pharyngitis, rheumatic fever, and glomerulonephritis related to infection by *Streptococcus pyogenes,* Groups C and G streptococci, *Corynebacterium diphtheriae*, or *Actinobacillus haemolyticum;* respiratory tract infections related to infection by *Mycoplasma pneumoniae, Legionella pneumophila, Streptococcus pneumoniae, Haemophilus influenzae*, or *Chlamydia* *pneumoniae;* blood and tissue infections, including endocarditis and osteomyelitis, caused by *S. aureus, S. haemolyticus, E. faecalis, E. faecium, E. durans,* including strains resistant to known antibacterials such as, but not limited to, beta-lactams, vancomycin, aminoglycosides, quinolones, chloramphenicol, tetracylines and macrolides; uncomplicated skin and soft tissue infections and abscesses, and puerperal fever related to infection by *Staphylococcus aureus,* coagulase-negative staphylococci (i.e., *S*. *epidermidis*, *S. hemolyticus*, etc.), *Streptococcus pyogenes*, *Streptococcus agalactiae,* Streptococcal groups C-F (minute-colony streptococci), viridans streptococci, *Corynebacterium minutissimum*, *Clostridium* spp., or *Bartonella henselae;* uncomplicated acute urinary tract infections related to infection by *Staphylococcus aureus,* coagulase-negative staphylococcal species, or *Enterococcus* spp.; urethritis and cervicitis; sexually transmitted diseases related to infection by *Chlamydia trachomatis, Haemophilus ducreyi, Treponema pallidum*, *Ureaplasma urealyticum,* or *Neiserria gonorrheae;* toxin diseases related to infection by *S*. *aureus* (food poisoning and toxic shock syndrome), or Groups A, B, and C streptococci; ulcers related to infection by *Helicobacter pylori;* systemic febrile syndromes related to infection by *Borrelia recurrentis;* Lyme disease related to infection by *Borrelia burgdorferi;* conjunctivitis, keratitis, and dacrocystitis related to infection by *Chlamydia trachomatis, Neisseria gonorrhoeae*, *S. aureus, S. pneumoniae, S. pyogenes, H. influenzae,* or *Listeria* spp.; disseminated *Mycobacterium avium* complex (MAC) disease related to infection by *Mycobacterium avium,* or *Mycobacterium intracellulare;* infections caused by *Mycobacterium tuberculosis, M. leprae*, *M. paratuberculosis, M. kansasii,* or M. *chelonei;* gastroenteritis related to infection by *Campylobacter jejuni;* intestinal protozoa related to infection by *Cryptosporidium* spp.; odontogenic infection related to infection by viridans streptococci; persistent cough related to infection by *Bordetella pertussis;* gas gangrene related to infection by *Clostridium perfringens* or *Bacteroides* spp.; and atherosclerosis or cardiovascular disease related to infection by *Helicobacter pylori* or *Chlamydia pneumoniae.* Bacterial infections and protozoal infections, and disorders related to such infections, which may be treated or prevented in animals include the following: bovine respiratory disease related to infection by *P. haemolytica, P. multocida, Mycoplasma bovis,* or *Bordetella* spp.; cow enteric disease related to infection by *E*. *coli* or protozoa (i.e., coccidia, cryptosporidia, etc.); dairy cow mastitis related to infection by S. *aureus, Strop. uberis, Streptococcus* agalactiae, *Streptococcus dysgalactiae, Klebsiella* spp., *Corynebacterium,* or *Enterococcus* spp.; swine respiratory disease related to infection by *A*. *pleuro., P. multocida,* or *Mycoplasma* spp.; swine enteric disease related to infection by *E*. *coli, Lawsonia intracellularis, Salmonella,* or *Serpulina hyodysinteriae*; cow footrot related to infection by *Fusobacterium* spp.; cow metritis related to infection by *E. coli*; cow hairy warts related to infection by *Fusobacterium necrophorum* or Bacteroides nodosus; cow pink-eye related to infection by *Moraxella bovis;* cow premature abortion related to infection by protozoa (i.e. neosporium); urinary tract infection in dogs and cats related to infection by *E*. *coli;* skin and soft tissue infections in dogs and cats related to infection by *S. epidermidis*, *S*. *intermedius,* coagulase neg. *Staphylococcus* or *P*. *multocida;* and dental or mouth infections in dogs and cats related to infection by *Alcaligenes* spp., *Bacteroides* spp., *Clostridium* spp., *Enterobacter* spp., *Eubacterium, Peptostreptococcus, Porphyromonas*, or *Prevotella*. Other bacterial infections and protozoal infections, and disorders related to such infections, which may be treated or prevented in accord with the method of the present invention are referred to in J. P. Sanford et al., "The Sanford Guide To Antimicrobial Therapy," 26th Edition, (Antimicrobial Therapy, Inc., 1996).

The term "halo", as used herein, unless otherwise indicated, includes fluoro, chloro, bromo or iodo. Preferred halo groups are fluoro, chloro and bromo.

The term "alkyl", as used herein, unless otherwise indicated, includes saturated monovalent hydrocarbon radicals having cyclic, straight and/or branched moieties. It is to be understood that to include cyclic moieties, the alkyl group must include at least 3 carbon atoms.

The term "alkenyl", as used herein, unless otherwise indicated, includes alkyl groups as defined above having at least one carbon-carbon double bond at some point in the alkyl chain.

The term "alkynyl", as used herein, unless otherwise indicated, includes alkyl groups as defined above having at least one carbon-carbon triple bond at some point in the alkyl chain.

The term "cycloalkylene", as used herein, unless otherwise indicated, includes divalent cycloalkyl groups.

The term "aryl", as used herein, unless otherwise indicated, includes an organic radical derived from an aromatic hydrocarbon by removal of one hydrogen, such as phenyl or naphthyl.

The term "4-10 membered heterocyclic", as used herein, unless otherwise indicated, includes aromatic and non-aromatic heterocyclic groups containing one or more heteroatoms each selected from O, S and N, wherein each heterocyclic group has from 4-10 atoms in its ring system. Non-aromatic heterocyclic groups include groups having only 4 atoms in their ring system, but aromatic heterocyclic groups must have at least 5 atoms in their ring system. The heterocyclic groups include benzo-fused ring systems and ring systems substituted with one or more oxo moieties. An example of a 4 membered heterocyclic group is azetidinyl (derived from azetidine). An example of a 5 membered heterocyclic group is thiazolyl and an example of a 10 membered heterocyclic group is quinolinyl. Examples of non-aromatic heterocyclic groups are pyrrolidinyl, tetrahydrofuranyl, tetrahydrothienyl, tetrahydropyranyl, tetrahydrothiopyranyl, piperidino, morpholino, thiomorpholino, thioxanyl, piperazinyl, azetidinyl, oxetanyl, thietanyl, homopiperidinyl, oxepanyl, thiepanyl, oxazepinyl, diazepinyl, thiazepinyl, 1,2,3,6-tetrahydropyridinyl, 2-pyrrolinyl, 3-pyrrolinyl, indolinyl, 2H-pyranyl, 4H-pyranyl, dioxanyl, 1,3-dioxolanyl, pyrazolinyl, dithianyl, dithiolanyl, dihydropyranyl, dihydrothienyl, dihydrofuranyl, pyrazolidinyl, imidazolinyl, imidazolidinyl, 3-azabicyclo[3.1.0]hexanyl, 3-azabicyclo[4.1.0]heptanyl, 3H-indolyl and quinolizinyl. Examples of aromatic heterocyclic groups are pyridinyl, imidazolyl, pyrimidinyl, pyrazolyl, triazolyl, pyrazinyl, tetrazolyl, furyl, thienyl, isoxazolyl, thiazolyl, oxazolyl, isothiazolyl, pyrrolyl, quinolinyl, isoquinolinyl, indolyl, benzimidazolyl, benzofuranyl, cinnolinyl, indazolyl, indolizinyl, phthalazinyl, pyridazinyl, triazinyl, isoindolyl, pteridinyl, purinyl, oxadiazolyl, thiadiazolyl, furazanyl, benzofurazanyl, benzothiophenyl, benzothiazolyl, benzoxazolyl, quinazolinyl, quinoxalinyl, naphthyridinyl, and furopyridinyl. The foregoing groups, as derived from the compounds listed above, may be C-attached or N-attached where such is possible. For instance, a group derived from pyrrole may be pyrrol-1-yl(N-attached) or pyrrol-3-yl(C-attached).

The phrase "pharmaceutically acceptable salt(s)", as used herein, unless otherwise indicated, includes salts of acidic or basic groups which may be present in the compounds of formula **1**. The compounds of formula **1** that are basic in nature are capable of forming a wide variety of salts with various inorganic and organic acids. The acids that may be used to prepare pharmaceutically acceptable acid addition salts of such basic compounds of formula **1** are those that form non-toxic acid addition salts, i.e., salts containing pharmacologically acceptable anions, such as the hydrochloride, hydrobromide, hydroiodide, nitrate, sulfate, bisulfate, phosphate, acid phosphate, isonicotinate, acetate, lactate, salicylate, citrate, acid citrate, tartrate, pantothenate, bitartrate, ascorbate, succinate, maleate, gentisinate, fumarate, gluconate, glucaronate, saccharate, formate, benzoate, glutamate, methanesulfonate, ethanesulfonate, benzenesulfonate, p-toluenesulfonate and pamoate [i.e., 1,1'-methylene-bis-(2-hydroxy-3-naphthoate)] salts.

Those compounds of the formula **1** that are acidic in nature, are capable of forming base salts with various pharmacologically acceptable cations. Examples of such salts include the alkali metal or alkaline earth metal salts and particularly, the sodium and potassium salts.

The present invention also includes all radiolabelled forms of the compounds of formula **1**, and pharmaceutically acceptable salts thereof, wherein the radiolabel is selected from ³H, ¹¹C and ¹⁴C. Such radiolabelled compounds are useful as research or diagnostic tools.

Certain compounds of formula **1** may have asymmetric centers and therefore exist in different enantiomeric forms. This invention relates to the use of all optical isomers and stereoisomers of the compounds of formula **1** and mixtures thereof. In particular, the invention includes both the E and Z isomers of the -OR¹ group connected to the nitrogen of the oxime moiety at C-9 of the macrolide ring of formula **1**.

### Detailed Description of the Invention

The preparation of the compounds of the present invention is illustrated in the following Schemes.

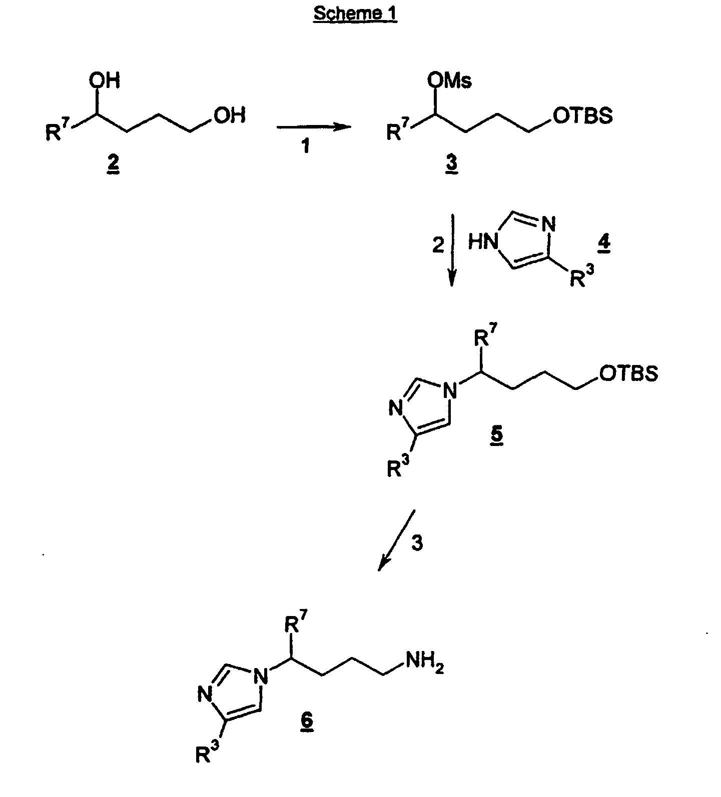

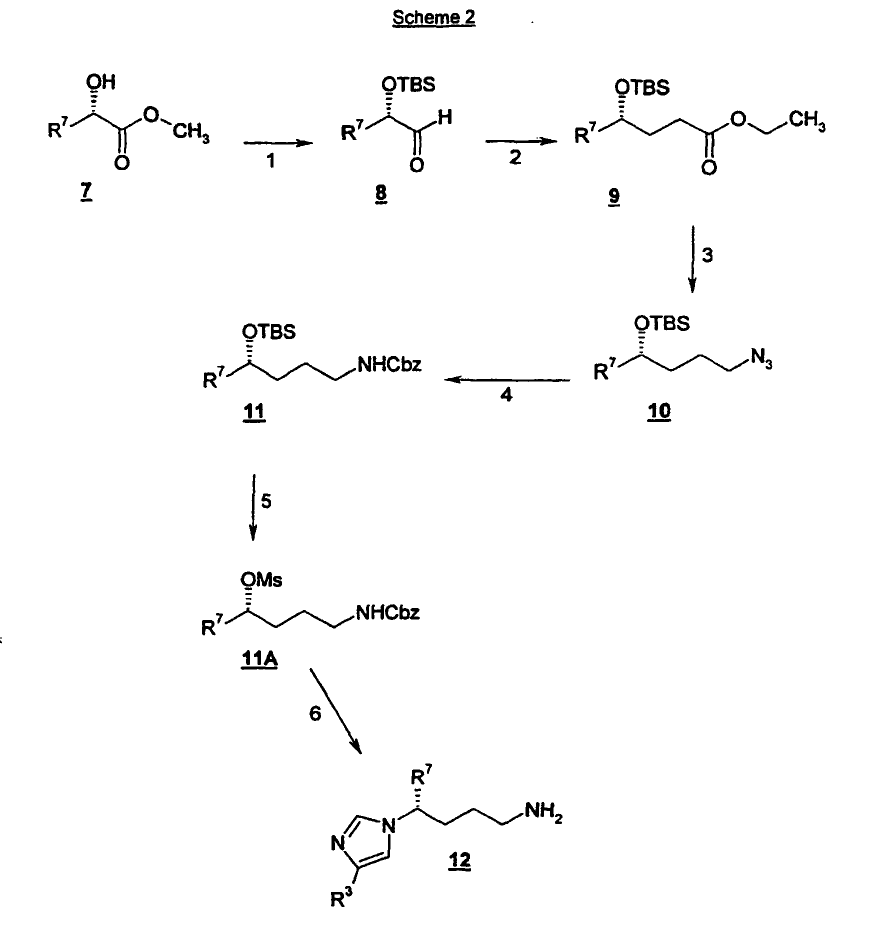

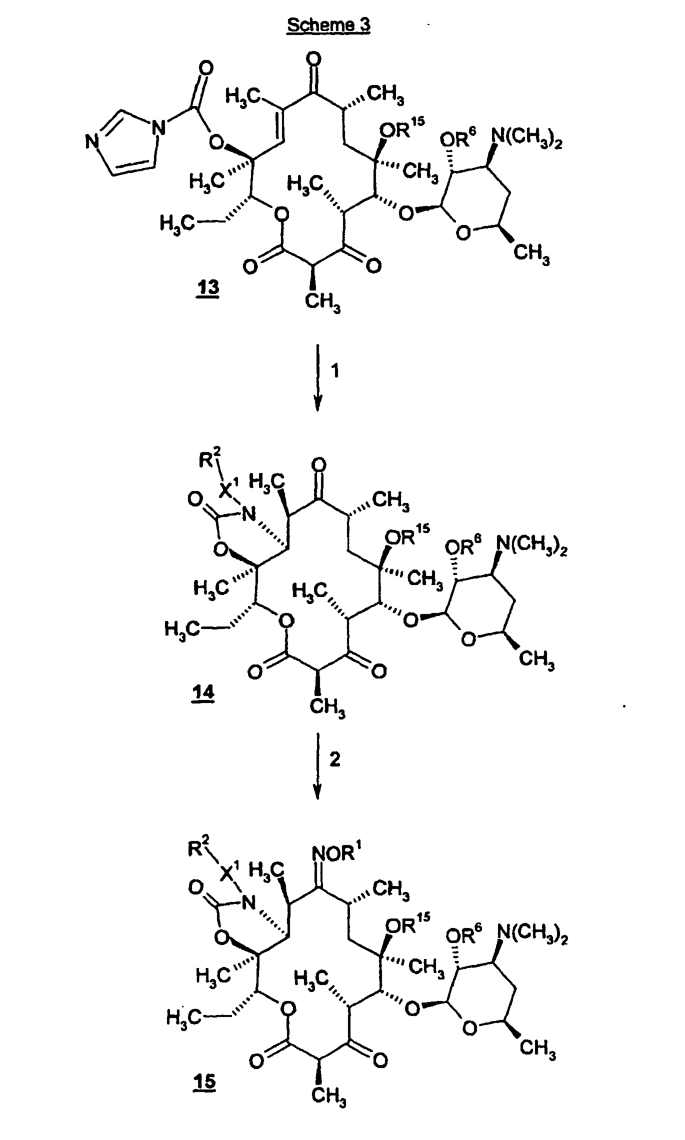

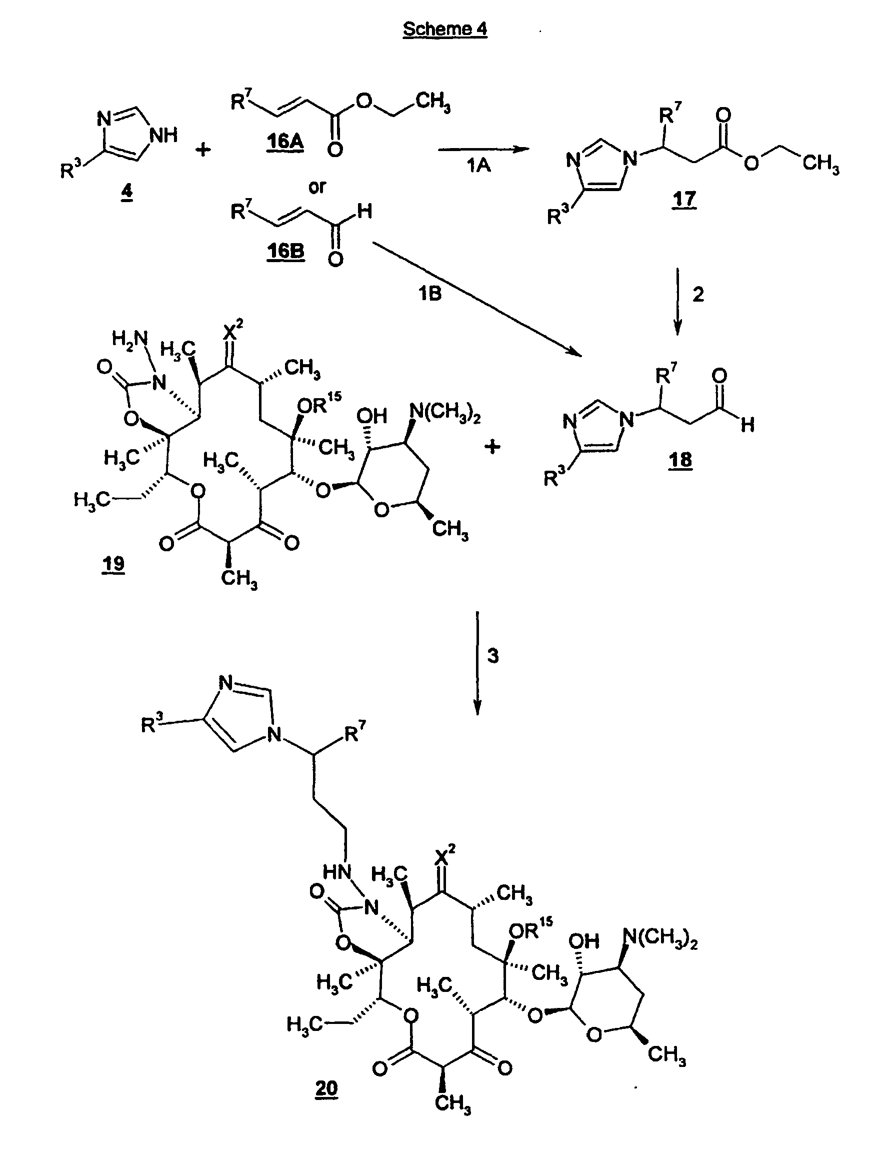

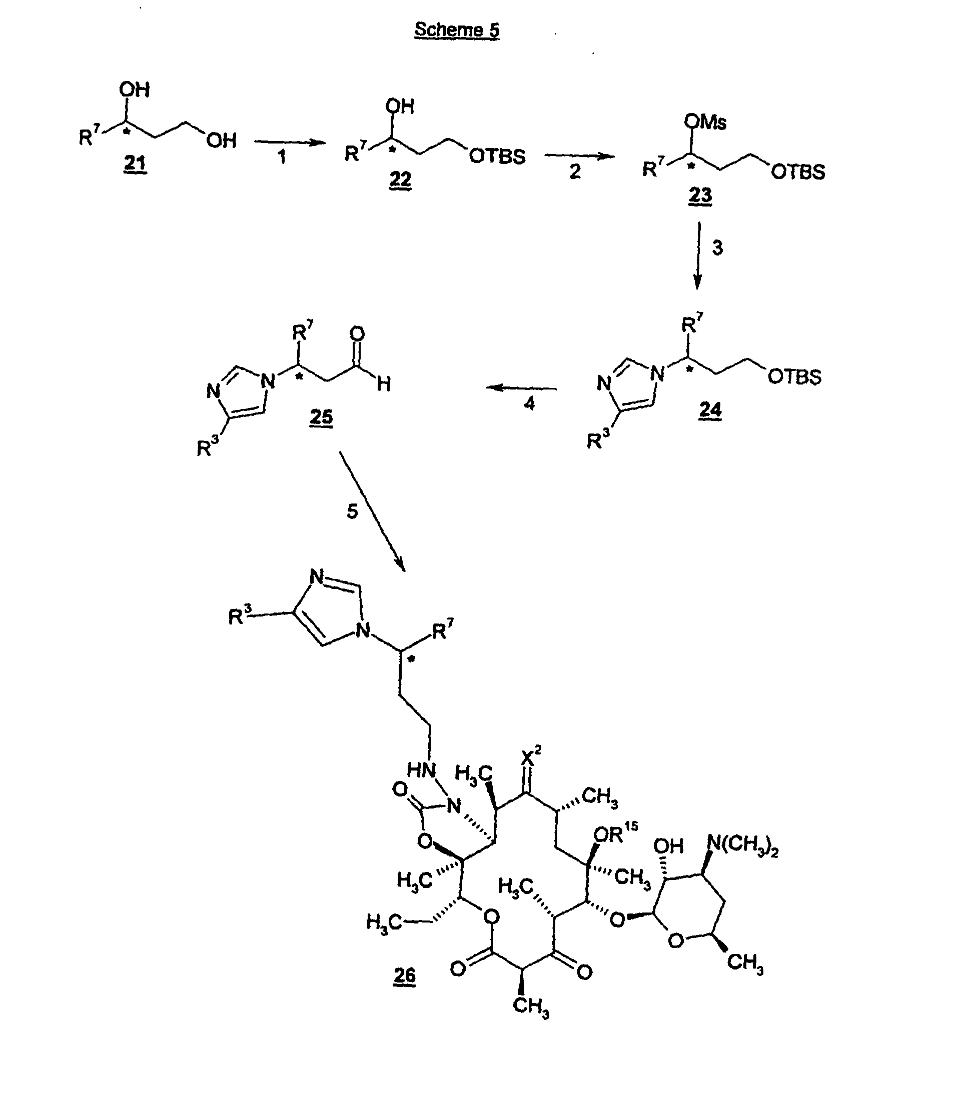

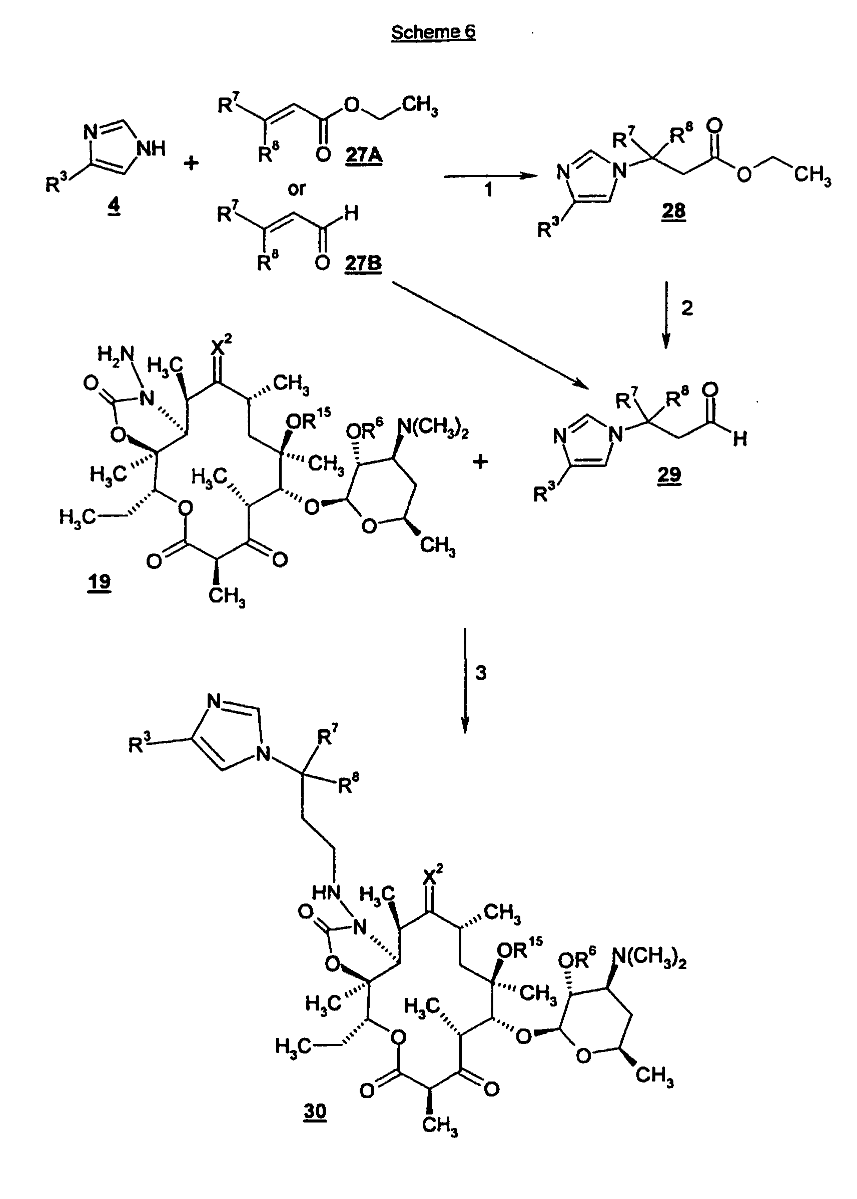

The preparation of the compounds of the present invention is illustrated in the above schemes. While the above Schemes illustrate the preparation of certain specific embodiments of the present invention, those skilled in the art would be able to prepare the full range of the claimed compounds using methods analogous to those illustrated above. The synthesis of racemic as well as enantiomerically pure side chains is illustrated in Schemes 1 and 2. In Scheme 1, the compound of formula **2,** in which R⁷ is an alkyl group as defined above, is commercially available or may be prepared according to methods familiar to those skilled in the art. Protection of the primary hydroxyl group as its *t*-butyldimethylsilyl ether (represented as OTBS in the compound of formula **3**) may be done by treatment of the compound of formula **2** with 1 equivalent of *t*-butyldimethylsilyl chloride and imidazole in N,N-dimethylformamide (DMF) at room temperature (approximately 20-25°C). Conversion of the secondary alcohol to the corresponding mesylate of formula **3** (in which Ms represents the mesylate moiety) may be done by reaction with methanesulfonyl chloride and triethylamine in dichloromethane at approximately -20°C. Displacement of the mesylate with the compound of formula **4**, wherein R³ is as defined above, to provide the compound of formula **5** may be accomplished by reaction of the compound of formula **4** with a base such as sodium hydride or potassium carbonate at approximately 80°C followed by addition of the mesylate compound of formula **3**. The compound of formula 4 may be prepared according to methods familiar to those skilled in the art, including one or more synthetic methods described in H. Bredereck, R. Gompper, H.G.v. Schuh, and G. Theilig, *Angew. Chem.,* **24**, 753 (1959). Deprotection of the silylether and conversion of the resultant alcohol to the amine of formula **6** may be achieved by the sequence: (1) treatment of the compound of formula **5** with tetrabutylammonium fluoride in tetrahydrofuran (THF) to provide the corresponding alcohol, (2) reaction of the alcohol with methanesulfonyl chloride and triethylamine to produce the mesylate, (3) displacement of the mesylate with sodium azide in DMF at room temperature to yield the primary azide, and (4) hydrogenation of the azide over palladium on carbon in methanol to provide the primary amine of formula **6**. This compound may be introduced into the macrolide structure as the side chain represented as -X¹-R² in the compound of formula **1** according to the methods described herein and according to one or more methods described in United States Patent 5,527,780 and PCT international application number PCT/IB98/00741, referred to above. Analogous side chains represented as -X¹-R² in the compound of formula **1** may be prepared in a similar manner.

While the side chain compounds prepared according to Scheme 1 are racemic, those prepared according to Scheme 2 are substantially enantiomerically pure. With reference to Scheme 2, the enantiomerically pure compound of the formula **7**, which is commercially available, such as S-(-)-methyl lactate, or prepared according to methods familiar to those skilled in the art, may be converted to its t-butyldimethylsilyl ether by treatment with t-butyldimethylchlorosilane in DMF in the presence of imidazole at a temperature ranging from 0°C to 40°C, preferably at room temperature. Reduction of this compound with di-isobutylaluminum hydride in toluene at approximately -70°C provides the aldehyde of formula **8** (wherein TBS represents t-butyldimethylsilyl). Wittig coupling of the compound of formula **8** with carbethoxymethylene triphenylphosphorane in benzene at a temperature ranging from 60°C to 80°C produces the corresponding unsaturated ester which may be hydrogenated over palladium in ethyl acetate to provide the ester of formula **9**. Reduction of the ester to the corresponding alcohol using lithium aluminum hydride in THF, conversion of the alcohol to the corresponding mesylate by treatment with methanesulfonyl chloride and triethylamine in dichloromethane at a temperature ranging from -20°C to 0°C, and, finally, displacement of the mesyl group with azide by reaction with sodium azide in DMF at room temperature affords the azide of formula **10**. Hydrogenation of the compound of formula **10** over palladium in a polar solvent, such as methanol, followed by reaction with benzylchloroformate provides the benzyloxycarbonyl amide of formula **11** (wherein Cbz represents benzyloxycarbonyl). Desilylation with tetra-n-butylammonium fluoride in THF followed by treatment with methanesulfonyl chloride and triethylamine at a temperature ranging from -20°C to 0°C produces the corresponding mesylate of formula **11A** (wherein Ms represents methanesulfonyl). Reaction of the compound of formula **11A** with a compound of the formula **4** (which is illustrated in Scheme 1) and sodium hydride in dry DMF at a temperature ranging from 20°C to 100°C, followed by deprotection by hydrogenation over a palladium catalyst in methanol at room temperature, provides a compound of the formula **12**. An example of a compound corresponding to formula **12** is (R)-4-(4-pyridin-3-yl-imidazol-1-yl)-pentylamine. Following the same procedures outlined above except starting with a compound having the opposite stereochemical orientation with respect to the hydroxy group, such as R-(+)-methyl lactate, provides a compound corresponding to the compound of formula **12** except the stereochemical orientation of the R⁷ group is opposite to that illustrated for the compound of formula **12**. An example of such a compound is (S)-4-(4-pyridin-3-yl-imidazol-1-yl)-pentylamine.

The synthesis of the final ketolide is illustrated in Scheme 3. The compound of formula **13**, wherein R⁶ is acetyl, may be prepared as described in United States patent 5,543,400 (issued August 6, 1996). In general, the intermediate compound of formula **14** may be prepared as described in United States patent 5,543,400, PCT international application number PCT/IB98/00741 and United States patent 5,527,780, each of which is referred to above, and also United Kingdom patent application number 2,288,174 (published October 11, 1995), and G. Griesgraber *et al*., "3-Keto-11,12-carbazate Derivatives of 6-*O*-Methylerythromycin A," Journal of Antibiotics, 49(5), 465-477 (1996).

In step 1 of Scheme 3, compounds of the formula **14**, wherein R⁶ is H, X¹ is -CH₂- and R² and R³ are as defined above, may be prepared by treating a compound of the formula **13** with a compound of the formula H₂N-X¹-R², wherein X¹ is -CH₂- and R² is as defined above, in a solvent such as acetonitrile, DMF, THF, dimethoxy ethane or dimethylsulfoxide (DMSO), preferably acetonitrile, at a temperature within the range of about 50°C to 90°C, preferably about 80°C, for a period of about 4 to 16 hours. Compounds of the formula **14**, wherein X¹ is -NH- and R² is as defined above, can be prepared as described below in reference to schemes 4-6 and further as described in United Kingdom patent application number 2,288,174, referred to above. In step 2 of Scheme 3, compounds of the formula **15** may be prepared by treating a compound of the formula **14** with a compound of the formula R¹ONH₂•HCl or R¹ONH₂, wherein R¹ is as defined above, in the presence of an acid, such as Py•HCl (wherein Py denotes pyridine) or Et₃N·HCl (wherein Et denotes ethyl), in a polar solvent, preferably methanol, ethanol, or isopropyl alcohol, at a temperature within the range of about 65°C to 95°C for a period of about 10 hours to 6 days.

Scheme 4 illustrates the preparation of compounds of formula **1** wherein X¹ is -NH-and R² is as defined above. In particular, Scheme 4 illustrates an R² moiety wherein "n" is 2, although groups wherein "n" has other values may be used following an analogous procedure. In the compounds illustrated in Scheme 4, R⁷ is an alkyl group and R⁸ (not shown) is H. In step 1 of Scheme 4, a compound of formula **16A** is treated with a compound of formula **4** in THF at room temperature to provide the compound of formula **17**. Reduction with di-isobutylaluminum hydride in dichloromethane at approximately -70°C provides the aldehyde of formula **18**. In the alternative, compound **18** may be obtained by treating a compound of formula **16B** with a compound of formula **4** in the presence of an acid in THF or DMF at 20-100°C. The preferred acids are acetic acid, *p*-toluenesulfonic acid, and pyridinium *p*-toluenesulfonate. The compound of formula **19**, wherein X² is as defined above, may be prepared as described in PCT international application number PCT/IB98/00741 and United States patent 5,527,760, referred to above. Further, the synthesis of 11,12-cyclic carbazates analogous to the compounds of formula **19** is described in W.R. Baker, J.D. Clark, R.L. Stephens, and K.H. Kim, *J*. *Org. Chem.,* **53**, 2340 (1988). Condensation of the aldehyde of formula **18** with the compound of formula **19** in toluene at approximately 100°C followed by reduction of the resultant imine in methanol with sodium cyanoborohydride at 23°C gave rise to the compound of formula 20 wherein the product is racemic with respect to the chiral carbon to which R⁷ is attached.

Scheme 5 illustrates the preparation of compounds that are similar to those of formula 20 except the product is substantially enantiomerically pure with respect to the chiral carbon to which R⁷ is attached. This is indicated in structures **21** - **26** in Scheme 5 wherein the asterisk represents a specific stereoisomeric orientation (specifically, R or S) with respect to the carbon to which R⁷ is attached. In the compounds illustrated in Scheme 5, R⁷ is an alkyl group and R⁸ (not shown) is H. The synthesis of these compounds begins with chiral starting materials, which are illustrated here as R- or S-1,3-butanediol (the compound of formula **21**). Mono-silylation by reaction with 1 equivalent of *t*-butyldimethylsilyl chloride in DMF in the presence of imidazole at room temperature, about 23°C, for about 12 hours provides mono-silyl ether (the compound of formula 22 wherein TBS is *t*-butyldimethylsilyl). Mesylation by treatment with 1 equivalent of methanesulfonyl chloride and triethylamine in dichloromethane at approximately -20°C for approximately 40 minutes provides the corresponding mesylate of formula **23** (wherein Ms denotes the mesylate moiety). Displacement of the mesylate with a compound of formula **4** (illustrated in Scheme 1) in DMF with sodium hydride provides the compound of formula **24** with complete inversion of the stereochemistry of the alpha R⁷ group. Desilylation by reaction with tetra-t-butylammonium fluoride in THF followed by swem oxidation with oxalyl chloride and DMSO provides the chiral alpha-R⁷ imidazole propionaldehyde of formula **25**. Coupling of the aldehyde with the cyclic carbazate of formula **19**, as described in reference to Scheme 4 above, provides the R-R⁷ or S-R⁷ compound of formula **26**.

Scheme 6 illustrates the preparation of compounds of formula **26** which are similar to those of formula **20** except that both R⁷ and R⁸ are alkyl groups. The synthesis illustrated in Scheme 6 follows the same general steps and conditions of the synthesis illustrated in Scheme 4. As in Scheme 4, compound **29** may be obtained by reacting compounds **27A** and **4** to generate compound **28** which may be treated to obtain compound **29**, or by treating a compound of formula **27B** with a compound of formula **4** in the presence of an acid in THF or DMF at 20-100°C. The starting materials are either commercially available or they may be prepared according to synthetic methods familiar to those skilled In the art.

The compounds of the present invention may have asymmetric carbon atoms. Such diastereomeric mixtures can be separated into their individual diastereomers on the basis of their physical chemical differences by methods known to those skilled in the art, for example, by chromatography or fractional crystallization. Enantiomers can be separated by converting the enantiomeric mixtures into a diastereomeric mixture by reaction with an appropriate optically active compound (e.g., alcohol), separating the diastereomers and converting (e.g., hydrolyzing) the individual diastereomers to the corresponding pure enantiomers. All such isomers, including diastereomeric mixtures and pure enantiomers are considered as part of the invention.

The compounds of formula **1** that are basic in nature are capable of forming a wide variety of different salts with various inorganic and organic acids. Although such salts must be pharmaceutically acceptable for administration to animals, it is often desirable in practice to initially isolate the compound of formula I from the reaction mixture as a pharmaceutically unacceptable salt and then simply convert the latter back to the free base compound by treatment with an alkaline reagent and subsequently convert the latter free base to a pharmaceutically acceptable acid addition salt. The acid addition salts of the base compounds of this invention are readily prepared by treating the base compound with a substantially equivalent amount of the chosen mineral or organic acid in an aqueous solvent medium or in a suitable organic solvent, such as methanol or ethanol. Upon careful evaporation of the solvent, the desired solid salt is readily obtained. The desired acid salt can also be precipitated from a solution of the free base in an organic solvent by adding to the solution an appropriate mineral or organic acid.

Those compounds of the formula **1** that are acidic in nature are capable of forming base salts with various pharmacologically acceptable cations. Examples of such salts include the alkali metal or alkaline-earth metal salts and particularly, the sodium and potassium salts. These salts may be prepared by conventional techniques. The chemical bases which are used as reagents to prepare the pharmaceutically acceptable base salts of this invention are those which form non-toxic base salts with the acidic compounds of formula **1**. Such non-toxic base salts include those derived from such pharmacologically acceptable cations as sodium, potassium calcium and magnesium, etc. These salts can be prepared by treating the corresponding acidic compounds with an aqueous solution containing the desired pharmacologically acceptable cations, and then evaporating the resulting solution to dryness, preferably under reduced pressure. Alternatively, they may also be prepared by mixing lower alkanolic solutions of the acidic compounds and the desired alkali metal alkoxide together, and then evaporating the resulting solution to dryness in the same manner as before. In either case, stoichiometric quantities of reagents are preferably employed in order to ensure completeness of reaction and maximum yields of the desired final product.

The activity of the compounds of the present invention against bacterial and protozoa pathogens is demonstrated by the compound's ability to inhibit growth of defined strains of human (Assay I) or animal (Assays II and III) pathogens.

### Assay I

Assay I, described below, employs conventional methodology and interpretation criteria and is designed to provide direction for chemical modifications that may lead to compounds that circumvent defined mechanisms of macrolide resistance. In Assay I, a panel of bacterial strains is assembled to include a variety of target pathogenic species, including representatives of macrolide resistance mechanisms that have been characterized. Use of this panel enables the chemical structure/activity relationship to be determined with respect to potency, spectrum of activity, and structural elements or modifications that may be necessary to obviate resistance mechanisms. Bacterial pathogens that comprise the screening panel are shown in the table below. In many cases, both the macrolide-susceptible parent strain and the macrolide-resistant strain derived from it are available to provide a more accurate assessment of the compound's ability to circumvent the resistance mechanism. Strains that contain the gene with the designation of *ermA*/*ermB*/*ermC* are resistant to macrolides, lincosamides, and streptogramin B antibiotics due to modifications (methylation) of 23S rRNA molecules by an Erm methylase, thereby generally prevent the binding of all three structural classes. Two types of macrolide efflux have been described; *msrA* encodes a component of an efflux system in staphylococci that prevents the entry of macrolides and streptogramins while *mefA*/*E* encodes a transmembrane protein that appears to efflux only macrolides. Inactivation of macrolide antibiotics can occur and can be mediated by either a phosphorylation of the 2'-hydroxyl (*mph*) or by cleavage of the macrocyclic lactone (esterase). The strains may be characterized using conventional polymerase chain reaction (PCR) technology and/or by sequencing the resistance determinant. The use of PCR technology in this application is described in J. Sutcliffe et al., "Detection Of Erythromycin-Resistant Determinants By PCR", Antimicrobial Agents and Chemotherapy, 40(11), 2562-2566 (1996). The assay is performed in microtiter trays and interpreted according to Performance Standards for Antimicrobial Disk Susceptibility Tests - Sixth Edition; Approved Standard, published by The National Committee for Clinical Laboratory Standards (NCCLS) guidelines; the minimum inhibitory concentration (MIC) is used to compare strains. Compounds are initially dissolved in dimethylsulfoxide (DMSO) as 40 mg/ml stock solutions.

| Strain Designation | Macrolide Resistance Mechanism(s) |
|---|---|
| Staphylococcus aureus 1116 | susceptible parent |
| Staphylococcus aureus 1117 | ermB |
| Staphylococcus aureus 0052 | susceptible parent |
| Staphylococcus aureus 1120 | ermC |
| Staphylococcus aureus 1032 | msrA, mph, esterase |
| Staphylococcus hemolyticus 1006 | msrA, mph |
| Streptococcus pyogenes 0203 | susceptible parent |
| Streptococcus pyogenes 1079 | ermB |
| Streptococcus pyogenes 1062 | susceptible parent |
| Streptococcus pyogenes 1061 | ermB |
| Streptococcus pyogenes 1064 | ermB |
| Streptococcus agalactiae 1024 | susceptible parent |
| Streptococcus agalactiae 1023 | ermB |
| Streptococcus pneumoniae 1016 | susceptible |
| Streptococcus pneumoniae 1046 | ermB |
| Streptococcus pneumoniae 1095 | ermB |
| Streptococcus pneumoniae 1175 | mefE |
| Streptococcus pneumoniae 0085 | susceptible |
| Haemophilus influenzae 0131 | susceptible |
| Moraxella catarrhalis 0040 | susceptible |
| Moraxella catarrhalis 1055 | erythromycin intermediate resistance |
| Escherichia coli 0266 | susceptible |

Assay II is utilized to test for activity against *Pasteurella multocida* and Assay III is utilized to test for activity against *Pasteurella haemolytica*.

### Assay II

This assay is based on the liquid dilution method in microliter format. A single colony of *P. multocida* (strain 59A067) is inoculated into 5 ml of brain heart infusion (BHI) broth. The test compounds are prepared by solubilizing 1 mg of the compound in 125 µl of dimethylsulfoxide (DMSO). Dilutions of the test compound are prepared using uninoculated BHI broth. The concentrations of the test compound used range from 200 µg/ml to 0.098 µg/ml by two-fold serial dilutions. The *P*. *multocida* inoculated BHI is diluted with uninoculated BHI broth to make a 10⁴ cell suspension per 200 µl. The BHI cell suspensions are mixed with respective serial dilutions of the test compound, and incubated at 37°C for 18 hours. The minimum inhibitory concentration (MIC) is equal to the concentration of the compound exhibiting 100% inhibition of growth of P. multocida as determined by comparison with an uninoculated control.

### Assay III

This assay is based on the agar dilution method using a Steers Replicator. Two to five colonies isolated from an agar plate are inoculated into BHI broth and incubated overnight at 37°C with shaking (200 rpm). The next morning, 300 µl of the fully grown *P. haemolytica* preculture is inoculated into 3 ml of fresh BHI broth and is incubated at 37°C with shaking (200 rpm). The appropriate amounts of the test compounds are dissolved in ethanol and a series of two-fold serial dilutions are prepared. Two ml of the respective serial dilution is mixed with 18 ml of molten BHI agar and solidified. When the inoculated *P*. *haemolytica* culture reaches 0.5 McFarland standard density, about 5 µl of the *P*. *haemolytica* culture is inoculated onto BHI agar plates containing the various concentrations of the test compound using a Steers Replicator and incubated for 18 hours at 37°C. Initial concentrations of the test compound range from 100-200 µg/ml. The MIC is equal to the concentration of the test compound exhibiting 100% inhibition of growth of *P*. *haemolytica* as determined by comparison with an uninoculated control.

The in vivo activity of the compounds of formula **1** can be determined by conventional animal protection studies well known to those skilled in the art, usually carried out in mice.

Mice are allotted to cages (10 per cage) upon their arrival, and allowed to acclimate for a minimum of 48 hours before being used. Animals are inoculated with 0.5 ml of a 3 x 10³ CFU/ml bacterial suspension *(P. multocida* strain 59A006) intraperitoneally. Each experiment has at least 3 non-medicated control groups including one infected with 0.1X challenge dose and two infected with 1X challenge dose; a 10X challenge data group may also be used. Generally, all mice in a given study can be challenged within 30-90 minutes, especially if a repeating syringe (such as a Cornwall® syringe) is used to administer the challenge. Thirty minutes after challenging has begun, the first compound treatment is given. It may be necessary for a second person to begin compound dosing if all of the animals have not been challenged at the end of 30 minutes. The routes of administration are subcutaneous or oral doses. Subcutaneous doses are administered into the loose skin in the back of the neck whereas oral doses are given by means of a feeding needle. In both cases, a volume of 0.2 ml is used per mouse. Compounds are administered 30 minutes, 4 hours, and 24 hours after challenge. A control compound of known efficacy administered by the same route is included in each test. Animals are observed daily, and the number of survivors in each group is recorded. The *P*. *multocida* model monitoring continues for 96 hours (four days) post challenge.

The PD₅₀ is a calculated dose at which the compound tested protects 50% of a group of mice from mortality due to the bacterial infection which would be lethal in the absence of drug treatment.

The compounds of formula **1**, and the pharmaceutically acceptable salts and solvates thereof (hereinafter "the active compounds"), may be adminstered through oral, parenteral, topical, or rectal routes in the treatment or prevention of bacterial or protozoa infections. In general, these compounds are most desirably administered in dosages ranging from about 0.2 mg per kg body weight per day (mg/kg/day) to about 200 mg/kg/day in single or divided doses (i.e., from 1 to 4 doses per day), although variations will necessarily occur depending upon the species, weight and condition of the subject being treated and the particular route of administration chosen. However, a dosage level that is in the range of about 4 mg/kg/day to about 50 mg/kg/day is most desirably employed. Variations may nevertheless occur depending upon the species of mammal, fish or bird being treated and its individual response to said medicament, as well as on the type of pharmaceutical formulation chosen and the time period and interval at which such administration is carried out. In some instances, dosage levels below the lower limit of the aforesaid range may be more than adequate, while in other cases still larger doses may be employed without causing any harmful side effects, provided that such larger doses are first divided into several small doses for administration throughout the day.

The active compounds may be administered alone or in combination with pharmaceutically acceptable carriers or diluents by the routes previously indicated, and such administration may be carried out in single or multiple doses. More particularly, the active compounds may be administered in a wide variety of different dosage forms, i.e., they may be combined with various pharmaceutically acceptable inert carriers in the form of tablets, capsules, lozenges, troches, hard candies, powders, sprays, creams, salves, suppositories, jellies, gels, pastes, lotions, ointments, aqueous suspensions, injectable solutions, elixirs, syrups, and the like. Such carriers include solid diluents or fillers, sterile aqueous media and various non-toxic organic solvents, etc. Moreover, oral pharmaceutical compositions can be suitably sweetened and/or flavored. In general, the active compounds are present in such dosage forms at concentration levels ranging from about 5.0% to about 70% by weight.

For oral administration, tablets containing various excipients such as microcrystalline cellulose, sodium citrate, calcium carbonate, dicalcium phosphate and glycine may be employed along with various disintegrants such as starch (and preferably com, potato or tapioca starch), alginic acid and certain complex silicates, together with granulation binders like polyvinylpyrrolidone, sucrose, gelatin and acacia. Additionally, lubricating agents such as magnesium stearate, sodium lauryl sulfate and talc are often very useful for tabletting purposes. Solid compositions of a similar type may also be employed as fillers in gelatin capsules; preferred materials in this connection also include lactose or milk sugar as well as high molecular weight polyethylene glycols. When aqueous suspensions and/or elixirs are desired for oral adinistration, the active compound may be combined with various sweetening or flavoring agents, coloring matter or dyes, and, if so desired, emulsifying and/or suspending agents as well, together with such diluents as water, ethanol, propylene glycol, glycerin and various like combinations thereof.

For parenteral administration, solutions of an active compound in either sesame or peanut oil or in aqueous propylene glycol may be employed. The aqueous solutions should be suitably buffered (preferably pH greater than 8) if necessary and the liquid diluent first rendered isotonic. These aqueous solutions are suitable for intravenous injection purposes. The oily solutions are suitable for intraarticular, intramuscular and subcutaneous injection purposes. The preparation of all these solutions under sterile conditions is readily accomplished by standard pharmaceutical techniques will known to those skilled in the art.

Additionally, it is also possible to administer the active compounds of the present invention topically and this may be done by way of creams, jellies, gels, pastes, patches, ointments and the like, in accordance with standard pharmaceutical practice.

For administration to animals other than humans, such as cattle or domestic animals, the active compounds may be administered in the feed of the animals or orally as a drench composition.

The active compounds may also be adminstered In the form of liposome delivery systems, such as small unilamellar vesicles, large unilamellar vesicles and multilamellar vesicles. Liposomes can be formed from a variety of phospholipids, such as cholesterol, stearylamine or phosphatidylcholines.

The active compounds may also be coupled with soluble polymers as targetable drug carriers. Such polymers can include polyvinylpyrrolidone, pyran copolymer, polyhydroxypropylmethacrylamide phenyl, polyhydroxyethylaspartamide-phenol, or polyethyleneoxide-polylysine substituted with palmitoylresidues. Furthermore, the active compounds may be coupled to a class of biodegradable polymers useful in achieving controlled release of a drug, for example, polylactic acid, polyglycolic acid, copolymers of polylactic and polyglycolic acid, polyepsilon caprolactone, polyhydroxy butyric acid, polyorthoesters, polyacetals, polydihydropyrans, polycyanoacrylates and cross-linked or amphipathic block copolymers of hydrogels.

The Examples provided below illustrate specific embodiments of the invention, but the invention is not limited in scope to the Examples specifically exemplified.

The Examples provided below illustrate specific embodiments of the invention, but the invention is not limited in scope to the Examples specifically exemplified.

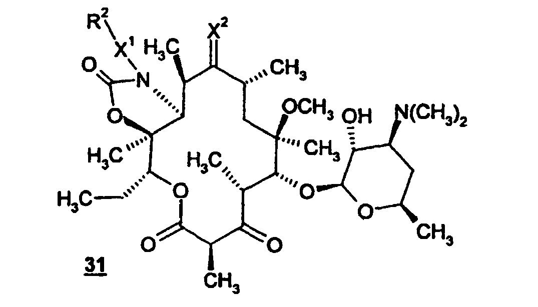

### Example 1 (not claimed)

To a solution of the allylic acylimidazole (0.95 g) of formula **13** in acetonitrile (6 ml) and THF (3 ml) was added (R/S)-4-(4-pyridin-3-yl-imidazol-1-yl)-pentylamine (0.624 g). The resulting solution was heated to reflux under nitrogen for 24 hours. Solvents were removed *in vacuo* and pumped to dryness. The resulting foam was re-suspended into methanol (10 ml) and heated under reflux for 5 hours. After cooling to room temperature (20-25°C), the mixture was poured into 5% sodium carbonate solution and extracted with dichloromethane (3x50 ml). Drying over potassium carbonate, filtration, concentration and purification by silica-gel chromatography (SGC) provided the compound of formula **31** illustrated above wherein -X¹-R² is (R/S)-4-(4-pyridin-3-yl-imidazol-1-yl)pentyl and X² is O. Yields ranged between 40 and 60%.

### Example 2 (not claimed)

Following the procedure described in Example 1, using (R)-4-(4-pyridin-3-yl-imidazol-1-yl)-pentylamine, the corresponding R-methyl isomer of formula **32** (below), wherein X² is O, Y is H, and X¹ is -CH₂-, was produced in similar yield.

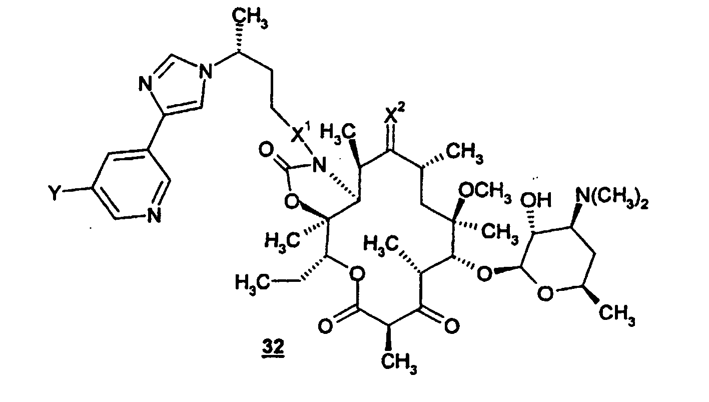

### Example 3 (not claimed)

Following the procedure described in Example 1, using (S)-4-(4-pyridin-3-yl-imidazol-1-yl)-pentylamine, the corresponding S-methyl isomer of formula **33** (below), wherein X² is O, Y is H, and X¹ is -CH₂-, was produced in similar yield.

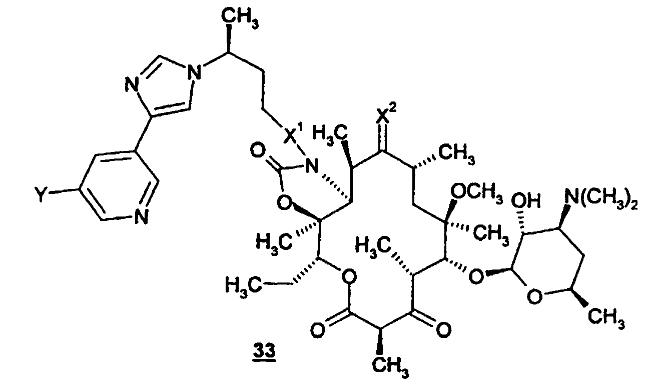

### Example 4 (not claimed)

Following the procedure described in Example 1, using (R)-4-[4-(5-fluoro)-pyridin-3-yl-imidazol-1-yl]-pentylamine (which may be prepared as described in E.P. Kyba, S. Liu, K. Chockalingam, B.R. Ready, J. Org. Chem., 53, 3513 (1988)), the corresponding R-methyl isomer of formula **32** (above), wherein X² is O, Y is fluoro, and X¹ is -CH₂-, is produced.

### Example 5 (not claimed)

Following the procedures described in Example 1, using (S)-4-[4-(5-fluoro)-pyridin-3-yl-imidazol-1-yl]-pentylamine (which may be prepared as described in E.P. Kyba, S. Liu, K. Chockalingam, B.R. Ready, J. Org. Chem., 53, 3513 (1988)), the corresponding S-methyl isomer of formula **33** (above), wherein X² is O, Y is fluoro, and X¹ is -CH₂-, is produced.

### Example 6 (not claimed)

The compound produced in Example 1 (100 mg) is dissolved in ethanol (2 ml). To this is added methoxylamine hydrochloride (50 mg). The resulting mixture is heated under reflux for 3 days. The mixture is diluted with water, and pH is adjusted to 9.5 with 1 N sodium hydroxide. Extraction with dichloromethane (3 x 25 ml), drying over potassium carbonate, concentration, and SGC purification results in the compound of formula **31**. illustrated above, wherein -X¹-R² is (R/S)-4-(4-pyridin-3-yl-imidazol-1-yl)pentyl and X² is =NOCH₃.

### Example 7 (not claimed)

Following the procedure of Example 6, using the compounds of Examples 2 through 5 as starting materials, compounds are obtained wherein -X¹-R² corresponds to that of Examples 2-5 and X² is =NOCH₃.

### Example 8 (not claimed)

To a solution of the compound of formula **19** (wherein X² is O)(see Scheme 4 above) (82 mg) in toluene (1 ml) was added 3-(R/S)-4-(4-pyridin-3-yl-imidazol-1-yl)-butyraldehyde (40 mg). The mixture was heated at 110°C for 12 hours. Solvent was removed and the resulting foam was re-suspended in methanol (5 ml). To it was added acetic acid (0.047 ml) and sodium cyanoborohydride (25 mg). After stirring for 12 hours, water was added and the pH was adjusted to 2 with 1N HCI. The mixture was stirred for 30 minutes and the pH was adjusted to 9.5 with 1N sodium hydroxide. Extraction with dichloromethane (3x25 ml), drying over potassium carbonate, filtration, concentration and SGC purification using 3% methanol-dichloromethane containing 0.3% concentrated ammonium hydroxide as eluent provided a compound of formula **31**, illustrated above, wherein -X¹-R² is (R/S)-3-(4-pyridin-3-yl-imidazol-1-yl)-butylamino and X² is O (67 mg).

### Example 9

Following the procedures described in Example 8, using the compound of formula **19** (wherein X² is =NOCH₃), the corresponding compound of of formula **31**, illustrated above, wherein -X¹-R² is (R/S)-3-(4-pyridin-3-yl-imidazol-1-yl)-butylamino and X² is =NOCH₃, was prepared in a yield similar to that found for the product of Example 8.

### Example 10 (not claimed)

Following the procedures described in Example 8, using 3-(R)-4-(4-pyridin-3-yl-imidazol-1-yl)-butyraldehyde and the compound of formula **19** wherein X² is =O as starting materials, the compound of of formula **32** (above), wherein X² is O, Y is H, and X¹ is NH, was obtained.

### Example 11

Following the procedures described in Example 8, using 3-(R)-4-(4-pyridin-3-yl-imidazol-1-yl)-butyraldehyde and the compound of formula **19** wherein X² is =NOCH₃ as starting materials, the compound of of formula **32** (above), wherein X² is =NOCH₃, Y is H, and X¹ is NH, was obtained.

### Example 12 (not claimed)

Following the procedures described in Example 8, using 3-(S)-4-(4-pyridin-3-yl-imidazol-1-yl)-butyraldehyde and the compound of formula **19** wherein X² is =O as starting materials, the compound of of formula **33** (above), wherein X² is O, Y is H, and X¹ is NH, was obtained.

### Example 13

Following the procedures described in Example 8, using 3-(S)-4-(4-pyridin-3-yl-imidazol-1-yl)-butyraldehyde and the compound of formula **19** wherein X² is =NOCH₃ as starting materials, the compound of of formula **33** (above), wherein X² is =NOCH₃, Y is H, and X¹ is NH, was obtained.

### Example 14

### 2-Methyl-3-(4-pyridin-3-yl-imidazol-1-yl)-propioaldehyde

To a solution of 4-pyridin-3-yl-imidazole (8.5 g) in THF (tetrahydrofuran) (293 mL) was added acetic acid (16.8 mL) and methacrolein (9.7 mL) and the resulting solution was heated under gentle reflux for 24 hours. Another 9.7 mL methacrolein was added and the solution was heated under reflux for another 24 hours. THF was then removed in vacuo and the residue was purified on a Flash 75 long column eluting with MeOH-CH₂Cl₂ to give the title compound as slightly yellow oil.
MS: m/z 215 (M+H).

### Example 15

### 3,3-Dimethyl-3-(4-pyridin-3-yl-imidazol-1-yl)-propioaldehyde

To a solution of 4-pyridin-3-yl-imidazole (1 g) in THF (34 mL) was added acetic acid (1.6 mL) and 3-methyl-2-butenal (3.3 mL) and the resulting solution was heated under gentle reflux for 24 hours. THF was then removed *in vacuo* and the residue was purified on a Flash 75 long column eluting with MeOH-CH₂Cl₂ to give the title compound as slightly yellow oil.
MS: m/z 230 (M+H).

### Example 15

### 2,2-Dimethyl-3-(4-pyridin-3-yl-imidazol-1-yl)-propyl acetate

To a solution of 4-pyridin-3-yl-imidazole (50 mg) in DMSO (methyl sulfoxide) (1.7 mL) was added powered KOH (57 mg) and 3-bromo-2,2-dimethylpropyl acetate and the resulting suspension was heated at 70°C for 48 hours. Water was added followed by CHCl₃, the aqueous layer was extracted with CHCl₃, the combined organic layers were washed with brine, dired over Na₂SO₄ and evaporated in vacuo. The redisue was purified by preparative TLC (10% MeOH-90% CH₂Cl₂) the title compound as slightly yellow oil.
MS: m/z 274 (M+H).

### Example 16

### 2,2-Dimethyl-3-(4-pyridin-3-yl-imidazol-1-yl)-propanol

To a solution of 2,2-Dimethyl-3-(4-pyridin-3-yl-imidazol-1-yl)-propyl acetate (20 mg) in methanol was added saturated solution of K₂CO₃ in metanol (1.5 mL) and the resulting solution was stirred at room temperature for 1 hour. MeOH was evaporated *in vacuo* and water was added followed by CH₂Cl₂. The aqueous layer was extracted with CHCl₃, the combined organic layers were washed with brine, dired over Na₂SO₄ and evaporated *in vacuo* to afford the title compound as a slightly yellow oil.
MS: m/z 232 (M+H).

### Example 17

### 2,2-Dimethyl-3-(4-pyridin-3-yl-imidazol-1-yl)-propioaldehyde

To a solution of 2,2-Dimethyl-3-(4-pyridin-3-yl-imidazol-1-yl)-propanol (11 mg) in CH₂Cl₂ was added Dess-Martin periodinane (41 mg) and the resulting solution was stirred at room temperature for 15 minutes. Saturated NaHCO₃ was added, the aqueous layer was extracted with CHCl₃, the combined organic layers were washed with brine, dired over Na₂SO₄ and evaporated in vacuo to afford the title compound as a slightly yellow oil.
MS: m/z 230 (M+H).

### Example 18

### 11-Deoxy-5-O-desosaminyl-11-(2,2-dimethyl-3-(4-pyridin-3-yl-imidazol-1-yl-propyl))hydrazo-6-O-methyl-3-oxoerythronolide A. 11,12-carbamate, 9-E-(O-methyl)oxime

To a solution of 11-deoxy-5-O-desosaminyl-11-hydrazo-6-O-methyl-3-oxoerythronolide A, 11,12-carbamate, 9-E-(O-methyl)oxime (24 mg) and 2,2-dimethyl-3-(4-pyridin-3-yl-imidazol-1-yl)-propioaldehyde (10 mg) in toluene (0.36 mL) was added acetic acid (6 uL) and the resulting solution was heated at 70°C for 24 hours. Toluene was evaporated *in* vacuo and the residue was then dissolved in methanol (0. 36 mL). Acetic acid (20 uL) was added to the above solution followed by NaBH₃CN (5 mg), and the resulting solution was stirred at room temperature for 1 hour. Saturated NaHCO₃ was added followed by CH₂Cl₂. Added. The aqueous layer was extracted with CH₂Cl₂ (3 times), the combined organic layers were washed with brine, dried over anhydrous MgSO₄, and concentrated *in* vacuo. The crude product was purified by preparative TLC (10% MeOH-1% NH₃·H₂O-89% CH₂Cl₂) to afford the title compound as a white solid.

¹H NMR (CDCl₃, 400 MHz) δ 3.63 (3H, s), 2.65 (3H, s), 2.28 (6H, s), 1.44 (3H, s), 1.34 (3H, s), 1.30 (3H, d, J = 6.8 Hz), 1.25 (3H, d, J = 7.6 Hz), 1.21 (3H, d, J = 6.0 Hz), 1.11 (3H, d, J = 7.2 Hz), 1.02 (3H, s), 0.97 (3H, d, J 7.2 Hz), 0.94 (3H, s), 0.74 (3H, t, J = 7.6 Hz).
S: m/z 870 (M+H).

### Example 19

### 11-Deoxy-5-O-desosaminyl-11-(2-methyl-3-(4-pyridin-3-yl-imidazol-1-yl-propyl))hydrazo-6-O-methyl-3-oxoerythronolide A, 11,12-carbamate, 9-E-(O-methyl)oxime

To a solution of 11-deoxy-5-O-desosaminyl-11-hydrazo-6-O-methyl-3-oxoerythronolide A, 11,12-carbamate, 9-E-(O-methyl)oxime (2.8 g) and 2-methyl-3-(4-pyridin-3-yl-imidazol-1-yl)-propioaldehyde (1.0 g) in toluene (0.36 mL) was added acetic acid (0.24 mL) and the resulting solution was heated at 70°C for 24 hours. Toluene was evaporated in vacuo and the residue was then dissolved in methanol (20 mL). Acetic acid (2.44 mL) was added to the above solution followed by NaBH₃CN (1.83 g), and the resulting solution was stirred at room temperature for 1 hour. Saturated NaHCO₃ was added followed by CH₂Cl₂. Added. The aqueous layer was extracted with CH₂Cl₂ (3 times), the combined organic layers were washed with brine, dried over anhydrous MgSO₄, and concentrated *in vacuo.* The crude product was purified by preparative HPLC by using Kromasil C18 column and 35% acetonitrile/64% 15mM ammonium acetate/1% trifluroacetic acid to give two isomers of the title compound.
One isomer: ¹H NMR (CDCl₃, 400 MHz) δ 3.71 (3H, s), 2.71 (3H, s), 2.25 (6H, s), 1.45 (3H, s), 1.39 (3H, s), 1.34 (3H, d, J = 6.8 Hz), 1.28 (3H, d, J = 7.6 Hz), 1.22 (3H, d, J = 6.0 Hz), 1.10 (3H, d, J = 6.8 Hz), 1.06 (3H, d, J = 6.8 Hz), 0.98 (3H, d, J = 6.8 Hz), 0.81 (3H, t, J = 7.2 Hz).
Another isomer: ¹H NMR (CDCl₃, 400 MHz) δ 3.73 (3H, s), 2.67 (3H, s), 2.25 (6H, s), 1.47 (3H, s), 1.38 (3H, s), 1.30 (3H, d, J = 6.8 Hz), 1.27 (3H, d, J = 7.2 Hz), 1.22 (3H, d, J = 6.0 Hz), 1.13 (3H, d, J = 6.8 Hz), 0.99 (3H, d, J = 6.8 Hz), 0.96 (3H, d, J = 6.8 Hz), 0.81 (3H, t, J = 6.8 Hz).

### Example 20

### 11-Deoxy-5-O-desosaminyl-11-(3,3-dimethyl-3-(4-pyridin-3-yl-imidazol-1-yl-propyl))hydrazo-6-O-methyl-3-oxoerythronolide A, 11,12-carbamate, 9-E-(O-methyl)oxime

To a solution of 11-deoxy-5-O-desosaminyl-11-hydrazo-6-O-methyl-3-oxoerythronolide A, 11,12-carbamate, 9-E-(O-methyl)oxime (257 mg) and 3,3-dimethyl-3-(4-pyridin-3-yl-imidazol-1-yl)-propioaldehyde (200 mg) in toluene (1.9 mL) was added acetic acid (0.09 mL) and the resulting solution was heated at room temperature for 24 hours. Toluene was evaporated in vacuo and the residue was then dissolved in methanol (2.6 mL). Acetic acid (0.3 mL) was added to the above solution followed by NaBH₃CN (49 mg), and the resulting solution was stirred at room temperature for 1 hour. Saturated NaHCO₃ was added followed by CH₂Cl₂. Added. The aqueous layer was extracted with CH₂Cl₂ (3 times), the combined organic layers were washed with brine, dried over anhydrous MgSO₄, and concentrated *in vacuo*. The crude product was purified by preparative TLC (10% MeOH-1% NH₃·H₂O-89% CH₂Cl₂) to afford the title compound as a white solid.

¹H NMR (CDCl₃, 400 MHz) δ 3.61 (3H, s), 2.55 (3H, s), 2.32 (6H, s), 1.62 (3H, s), 1.60 (3H, s), 1.42 (3H, s), 1.32 (3H, d, J = 6.8 Hz), 1.30 (3H, s), 1.24 (3H, d, J = 7.6 Hz), 1.21 (3H, d, J = 5.6 Hz), 1.02 (3H, J = 7.2 Hz), 0.93 (3H, d, J = 6.8 Hz), 0.81 (3H, t, J = 7.2 Hz).
MS: m/z 870 (M+H).

### Example 21

### 2-Methyl-3-(4-pyridin-3-yl-imidazol-1-yl)-propioaldehyde

To a solution of 4-pyridin-3-yl-imidazole (8.5 g) in THF (293 mL) was added acetic acid (16.8 mL) and methacrolein (9.7 mL) and the resulting solution was heated under gentle reflux for 24 hours. Another 9.7 mL methacrolein was added and the solution was heated under reflux for another 24 hours. THF was then removed *in vacuo* and the residue was purified on a Flash 75 long column eluting with MeOH-CH₂Cl₂ to give the title compound as slightly yellow oil.
MS: m/z 215 (M+H).

### Example 22

### 2,2-Dimethyl-3-(4-pyridin-3-yl-imidazol-1-yl)-propyl acetate

To a solution of 4-pyridin-3-yl-imidazole (50 mg) in DMSO (1.7 mL) was added powered KOH (57 mg) and 3-bromo-2,2-dimethylpropyl acetate and the resulting suspension was heated at 70°C for 48 hours. Water was added followed by CHCl₃, the aqueous layer was extracted with CHCl₃, the combined organic layers were washed with brine, dired over Na₂SO₄ and evaporated in vacuo. The residue was purified by preparative TLC (10% MeOH-90% CH₂Cl₂) the title compound as slightly yellow oil.
MS: m/z 274 (M+H).

### Example 23

### 2,2-Dimethyl-3-(4-pyridin-3-yl-imidazol-1-yl)-propanol

To a solution of 2,2-Dimethyl-3-(4-pyridin-3-yl-imldazol-1-yl)-propyl acetate (20 mg) in methanol was added saturated solution of K₂CO₃ in metanol (1.5 mL) and the resulting solution was stirred at room temperature for 1 hour. MeOH was evaporated *in vacuo* and water was added followed by CH₂Cl₂. The aqueous layer was extracted with CHCl₃, the combined organic layers were washed with brine, dried over Na₂SO₄ and evaporated in *vacuo* to afford the title compound as a slightly yellow oil.
MS: m/z 232 (M+H).

### Example 24

### 2,2-Dimethyl-3-(4-pyridin-3-yl-imidazol-1-yl)-propioaldehyde

To a solution of 2,2-Dimethyl-3-(4-pyridin-3-yl-imidazol-1-yl)-propanol (11 mg) in CH₂Cl₂ was added Dess-Martin periodinane (41 mg) and the resulting solution was stirred at room temperature for 15 minutes. Saturated NaHCO₃ was added, the aqueous layer was extracted with CHCl₃, the combined organic layers were washed with brine, dried over Na₂SO₄ and evaporated *in vacuo* to afford the title compound as a slightly yellow oil.
MS: m/z 230 (M+H).

### Example 25

### 11-Deoxy-5-O-desosaminyl-11-(2,2-dimethyl-3-(4-pyridin-3-yl-imidazol-1-yl)-propyl)hydrazo-6-O-methyl-3-oxoerythronolide A, 11,12-carbamate, 9-E-(O-methyl)oxime

To a solution of 11-deoxy-5-O-desosaminyl-11-hydrazo-6-O-methyl-3-oxoerythronolide A, 11,12-carbamate, 9-E-(O-methyl)oxime (24 mg) and 2,2-dimethyl-3-(4-pyridin-3-yl-imidazol-1-yl)-propioaldehyde (10 mg) in toluene (0.36 mL) was added acetic acid (6 uL) and the resulting solution was heated at 70°C for 24 hours. Toluene was evaporated in vacuo and the residue was then dissolved in methanol (0. 36 mL). Acetic acid (20 uL) was added to the above solution followed by NaBH₃CN (5 mg), and the resulting solution was stirred at room temperature for 1 hour. Saturated NaHCO₃ was added followed by CH₂Cl₂. Added. The aqueous layer was extracted with CH₂Cl₂ (3 times), the combined organic layers were washed with brine, dried over anhydrous MgSO₄, and concentrated *in vacuo.* The crude product was purified by preparative TLC (10% MeOH-1% NH₃•H₂O-89% CH₂Cl₂) to afford the title compound as a white solid.

¹H NMR (CDCl₃, 400 MHz) δ 3.63 (3H, s), 2.65 (3H, s), 2.28 (6H, s), 1.44 (3H, s), 1.34 (3H, s), 1.30 (3H, d, J = 6.8 Hz), 1.25 (3H, d, J = 7.6 Hz), 1.21 (3H, d, J = 6.0 Hz), 1.11 (3H, d, J = 7.2 Hz), 1.02 (3H, s), 0.97 (3H, d, J 7.2 Hz), 0.94 (3H, s), 0.74 (3H, t, J = 7.6 Hz).
MS: m/z 870 (M+H).

### Example 26

### 11-Deoxy-5-O-desosaminyl-11-(2-methyl-3-(4-pyridin-3-yl-imidazol-1-yl)-propyl)hydrazo-6-O-methyl-3-oxoerythronolide A, 11,12-carbamate, 9-E-(O-methyl)oxime

To a solution of 11-deoxy-5-O-desosaminyl-11-hydrazo-6-O-methyl-3-oxoerythronolide A, 11,12-carbamate, 9-E-(O-methyl)oxime (2.8 g) and 2-methyl-3-(4-pyridin-3-yl-imidazol-1-yl)-propioaldehyde (1.0 g) in toluene (0.36 mL) was added acetic acid (0.24 mL) and the resulting solution was heated at 70°C for 24 hours. Toluene was evaporated in vacuo and the residue was then dissolved in methanol (20 mL). Acetic acid (2.44 mL) was added to the above solution followed by NaBH₃CN (1.83 g), and the resulting solution was stirred at room temperature for 1 hour. Saturated NaHCO₃ was added followed by CH₂Cl₂. The aqueous layer was extracted with CH₂Cl₂ (3 times), the combined organic layers were washed with brine, dried over anhydrous MgSO₄, and concentrated *in vacuo*. The crude product was purified by preparative HPLC by using a Kromasil C18 column and 35% acetonitrile/64% 15mM ammonium acetate/1% trifluroacetic acid to give two isomers of the title compound.
One isomer: ¹H NMR (CDCl₃, 400 MHz) δ 3.71 (3H, s), 2.71 (3H, s), 2.25 (6H, s), 1.45 (3H, s), 1.39 (3H, s), 1.34 (3H, d, J = 6.8 Hz), 1.28 (3H, d, J = 7.6 Hz), 1.22 (3H, d, J = 6.0 Hz), 1.10 (3H, d, J = 6.8 Hz), 1.06 (3H, d, J = 6.8 Hz), 0.98 (3H, d, J = 6.8 Hz), 0.81 (3H, t, J = 7.2 Hz).
Second isomer: ¹H NMR (CDCl₃, 400 MHz) δ 3.73 (3H, s), 2.67 (3H, s), 2.25 (6H, s), 1.47 (3H, s), 1.38 (3H, s), 1.30 (3H, d, J = 6.8 Hz), 1.27 (3H, d, J = 7.2 Hz), 1.22 (3H, d, J = 6.0 Hz), 1.13 (3H, d, J = 6.8 Hz), 0.99 (3H, d, J = 6.8 Hz), 0.96 (3H, d, J = 6.8 Hz), 0.81 (3H, t, J = 6.8 Hz).

Specific compounds that are part of the present invention and have been or can be made according to the methods provided above, include the following:
11-Deoxy-5-O-desosaminyl-11-(3,3-dimethyl-3-(4-pyridin-3-yl-imidazol-1-yl)-propyl)hydrazo-6-O-methyl-3-oxoerythronolide A, 11,12-carbamate, 9-E-(O-methyl)oxime;
11-Deoxy-5-O-desosaminyl-11-(3(R)-methyl-3-(4-pyridin-3-yl-imidazol-1-yl)-propyl)hydrazo-6-O-methyl-3-oxoerythronolide A, 11, 12-carbamate, 9-E-(O-methyl)oxime;
11-Deoxy-5-O-desosaminyl-11-(3(R)-ethyll-3-(4-pyridin-3-yl-imidazol-1-yl)-propyl)hydrazo-6-O-methyl-3-oxoerythronolide A, 11,12-carbamate, 9-E-(O-methyl)oxime;
11-Deoxy-5-O-desosaminyl-11-(3(S)-methyl-3-(4-pyridin-3-yl-imidazol-1 -yl)-propyl)hydrazo-6-O-methyl-3-oxoerythronolide A, 11,12-carbamate, 9-E-(O-methyl)oxime;
11-Deoxy-5-O-desosaminyl-11-(3(S)-ethyl-3-(4-pyridin-3-yl-imidazol-1-yl)-propyl)hydrazo-6-O-methyl-3-oxoerythronolide A, 11,12-carbamate, 9-E-(O-methyl)oxime;
11-Deoxy-5-O-desosaminyl-11-(2-(1-(4-pyridin-3-yl-imidazol-1-yl)-cyclopropyl)-ethyl)hydrazo-6-O-methyl-3-oxoerythronolide A, 11,12-carbamate, 9-E-(O-methyl)oxime;
11-Deoxy-5-O-desosaminyl-11-(2-(1-(4-pyridin-3-yl-imidazol-1-yl)-cyclobutyl)-ethyl)hydrazo-6-O-methyl-3-oxoerythronolide A, 11,12-carbamate, 9-E-(O-methyl)oxime;
11-Deoxy-5-O-desosaminyl-11-(2,2-dimethyl-3-(4-pyridin-3-yl-imidazol-1-yl)-propyl)hydrazo-6-O-methyl-3-oxoerythronolide A, 11,12-carbamate, 9-E-(O-methyl)oxime;
11-Deoxy-5-O-desosaminyl-11-(2,2-diethyl-3-(4-pyridin-3-yl-imidazol-1-yl)-propyl)hydrazo-6-O-methyl-3-oxoerythronolide A, 11,12-carbamate, 9-E-(O-methyl)oxime;
11-Deoxy-5-O-desosaminyl-11-(2,2-difluro-3-(4-pyridin-3-yl-imidazol-1-yl)-propyl)hydrazo-6-O-methyl-3-oxoerythronolide A, 11,12-carbamate, 9-E-(O-methyl)oxime;
11-Deoxy-5-O-desosaminyl-11-(2,2-dichloro-3-(4-pyridin-3-yl-imidazol-1-yl)-propyl)hydrazo-6-O-methyl-3-oxoerythronolide A, 11,12-carbamate, 9-E-(O-methyl)oxime;
11-Deoxy-5-O-desosaminyl-11-(2,2-dibromo-3-(4-pyridin-3-yl-imidazol-1-yl)-propyl)hydrazo-6-O-methyl-3-oxoerythronolide A, 11,12-carbamate, 9-E-(O-methyl)oxime;
11-Deoxy-5-O-desosaminyl-11-(1-(4-pyridin-3-yl-imidazol-1-yl-methyl)-cyclopropyl)-methyl)hydrazo-6-O-methyl-3-oxoerythronolide A, 11,12-carbamate, 9-E-(O-methyl)oxime;
11-Deoxy-5-O-desosaminyl-11-(1-(4-pyridin-3-yl-imidazol-1-yl-methyl)-cyclobutyl)-methyl)hydrazo-6-O-methyl-3-oxoerythronolide A, 11,12-carbamate, 9-E-(O-methyl)oxime;
11-Deoxy-5-O-desosaminyl-11-(2(R)-methyl-3-(4-pyridin-3-yl-imidazol-1-yl)-propyl)hydrazo-6-O-methyl-3-oxoerythronolide A, 11,12-carbamate, 9-E-(O-methyl)oxime;
11-Deoxy-5-O-desosaminyl-11-(2(R)-ethyl-3-(4-pyridin-3-yl-imidazol-1-yl)-propyl)hydrazo-6-O-methyl-3-oxoerythronolide A, 11,12-carbamate, 9-E-(O-methyl)oxime;
11-Deoxy-5-O-desosaminyl-11-(2(S)-methyl-3-(4-pyridin-3-yl-imidazol-1-yl)-propyl)hydrazo-6-O-methyl-3-oxoerythronolide A, 11,12-carbamate, 9-E-(O-methyl)oxime;
11-Deoxy-5-O-desosaminyl-11-(2(S)-ethyl-3-(4-pyridin-3-yl-imidazol-1-yl)-propyl)hydrazo-6-O-methyl-3-oxoerythronolide A, 11,12-carbamate, 9-E-(O-methyl)oxime;
11-Deoxy-5-O-desosaminyl-11-(2(R)-fluro-3-(4-pyridin-3-yl-imidazol-1-yl)-propyl)hydrazo-6-O-methyl-3-oxoerythronolide A, 11,12-carbamate, 9-E-(O-methyl)oxime;
11-Deoxy-5-O-desosaminyl-11-(2(S)-flurol-3-(4-pyridin-3-yl-imidazol-1-yl)-propyl)hydrazo-6-O-methyl-3-oxoerythronolide A, 11,12-carbamate, 9-E-(O-methyl)oxime;
and the pharmaceutically acceptable salts, prodrugs and solvates of the foregoing compounds.

Specific compounds of the present invention include
11-Deoxy-5-O-desosaminyl-11-(2-(1 -(4-pyridin-3-yl-imidazol-1-yl)-cyclopropyl)-ethyl)hydrazo-6-O-methyl-3-oxoerythronolide A, 11,12-carbamate, 9-E-(O-methyl)oxime;
11-Deoxy-5-O-desosaminyl-11-(2-(1-(4-pyridin-3-yl-imidazol-1-yl)-cyclobutyl)-ethyl)hydrazo-6-O-methyl-3-oxoerythronolide A, 11,12-carbamate, 9-E-(O-methyl)oxime;
11-Deoxy-5-O-desosaminyl-11-(2,2-dimethyl-3-(4-pyridin-3-yl-imidazol-1-yl)-propyl)hydrazo-6-O-methyl-3-oxoerythronolide A, 11,12-carbamate, 9-E-(O-methyl)oxime;
11-Deoxy-5-O-desosaminyl-11-(2,2-diethyl-3-(4-pyridin-3-yl-imidazol-1-yl)-propyl)hydrazo-6-O-methyl-3-oxoerythronolide A, 11,12-carbamate, 9-E-(O-methyl)oxime;
11-Deoxy-5-O-desosaminyl-11-(2,2-difluoro-3-(4-pyridin-3-yl-imidazol-1-yl)-propyl)hydrazo-6-O-methyl-3-oxoerythronolide A, 11,12-carbamate, 9-E-(O-methyl)oxime;
11-Deoxy-5-O-desosaminyl-11-(2,2-dichloro-3-(4-pyridin-3-yl-imidazol-1-yl)-propyl)hydrazo-6-O-methyl-3-oxoerythronolide A, 11,12-carbamate, 9-E-(O-methyl)oxime;
11-Deoxy-5-O-desosaminyl-11-(2,2-dibromo-3-(4-pyridin-3-yl-imidazol-1-yl)-propyl)hydrazo-6-O-methyl-3-oxoerythronolide A, 11,12-carbamate, 9-E-(O-methyl)oxime;
11-Deoxy-5-O-desosaminyl-11-(1-(4-pyridin-3-yl-imidazol-1-yl-methyl)-cyclopropyl)-methyl)hydrazo-6-O-methyl-3-oxoerythronolide A, 11,12-carbamate, 9-E-(O-methyl)oxime;
11-Deoxy-5-O-desosaminyl-11-(1-(4-pyridin-3-yl-imidazol-1-yl-methyl)-cyclobutyl)-methyl)hydrazo-6-O-methyl-3-oxoerythronolide A, 11,12-carbamate, 9-E-(O-methyl)oxime;
11-Deoxy-5-O-desosaminyl-11-(2-(R)-methyl-3-(4-pyridin-3-yl-imidazol-1-yl)-propyl)hydrazo-6-O-methyl-3-oxoerythronolide A, 11,12-carbamate, 9-E-(O-methyl)oxime;
11-Deoxy-5-O-desosaminyl-11-(2-(R)-ethyl-3-(4-pyridin-3-yl-imidazol-1-yl-propyl))hydrazo-6-O-methyl-3-oxoerythronolide A, 11,12-carbamate, 9-E-(O-methyl)oxime;
11-Deoxy-5-O-desosaminyl-11-(2-(S)-methyl-3-(4-pyridin-3-yl-imidazol-1-yl)-propyl)hydrazo-6-O-methyl-3-oxoerythronolide A, 11,12-carbamate, 9-E-(O-methyl)oxime;
11-Deoxy-5-O-desosaminyl-11-(2-(S)-ethyl-3-(4-pyridin-3-yl-imidazol-1-yl)-propyl)hydrazo-6-O-methyl-3-oxoerythronolide A, 11,12-carbamate, 9-E-(O-methyl)oxime;
11-Deoxy-5-O-desosaminyl-11-(2-(R)-fluoro-3-(4-pyridin-3-yl-imidazol-1-yl)-propyl)hydrazo-6-O-methyl-3-oxoerythronolide A, 11,12-carbamate, 9-E-(O-methyl)oxime;
11-Deoxy-5-O-desosaminyl-11-(2-(S)-fluoro-3-(4-pyridin-3-yl-imidazol-1-yl-propyl))hydrazo-6-O-methyl-3-oxoerythronolide A, 11,12-carbamate, 9-E-(O-methyl)oxime;
and the pharmaceutically acceptable salts, prodrugs and solvates of the foregoing compounds.

## Claims

1. A compound of the formula 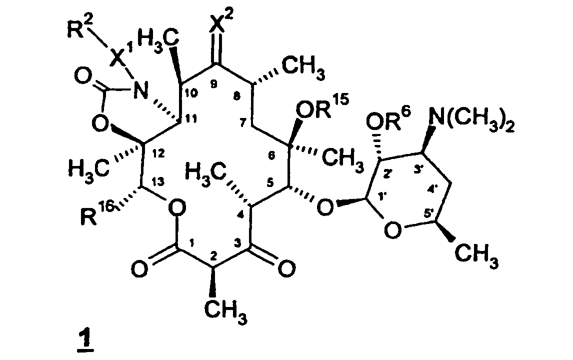 or a pharmaceutical acceptable salt or solvate thereof, wherein:
X¹ is -NR⁴-;
X² is =NOR¹:
R¹ is H, methyl or ethyl;
R² is -C(R⁷)(R⁸)C(R⁹)(R¹⁰)C(R¹¹)(R¹²)(4-10 membered heterocyclic) or -(CR⁷R⁸)ᵣ(C₃₋C₇ cycloalkylene)(CR⁹R¹⁰)_{q}(4-10 membered heterocyclic), wherein q and r are each integers independently ranging from 0 to 2, and the heterocyclic moiety of the foregoing groups is substituted by -(CR⁴R⁵)ₜ(4-10 membered heterocyclic) or -(CR⁴R⁵)ₜ(C₆-C₁₀ aryl) wherein t is an integer from 0 to 5, and the aryl and heterocyclic moieties of each of the foregoing groups are optionally substituted by 1 to 4 R³ groups;
each R³ is independently selected from the group consisting of halo, cyano, nitro, trifluoromethyl, trifiuoromethoxy, azido, -C(O)R⁵, -C(O)OR⁵, -OC(O)R⁵, -NR⁵C(O)R⁴, -C(O)NR⁴R⁵, -NR⁴R⁵, hydroxy, C₁-C₆ alkyl, C₂-C₆ alkenyl. C₂-C₆ alkynyl. C₆-C₁₀ aryl, 4-10 membered heterocyclic, and C₁-C₆ alkoxy;
each R⁴ and R⁵ is independently selected from H and C₁-C₆ alkyl;
R⁶ is H or -C(O)(C₁-C₆ alkyl);
R⁷, R⁸, R⁹, R¹⁰, R¹¹, R¹², R¹³ and R¹⁴ are each independently selected from H. halo and C₁-C₆ alkyl, provided that (a) where X¹ is -NR⁴- and R² is -C(R⁷)(R⁸)C(R⁹)(R¹⁰)C(R¹¹)(R¹²)(4-10 membered heterocyclic), at least one of R⁷, R⁸, R⁹, R¹⁰, R¹¹, and R¹² is halo or C₁-C₆ alkyl;
or R⁷ and R⁸ are taken together with the carbon to which each is attached to form a C₃-C₇ cycloalkyl group and R⁹, R¹⁰, R¹¹, R¹², R¹³ and R¹⁴ are each independently selected from H, halo and C₁-C₆ alkyl;
or R⁹ and R¹⁰ are taken together with the carbon to which each is attached to form a C₃-C₆ cycloalkyl group and R⁷, R⁸, R¹¹, R¹², R¹¹ and R¹⁴ are each independently selected from H, halo and C₁-C₆ alkyl;
or R¹¹ and R¹² are taken together with the carbon to which each is attached to form a C₃-C₇ cycloalkyl group and R⁷, R⁸, R⁹, R¹⁰, R¹³ and R¹⁴ are each independently selected from H, halo and C₁-C₆ alkyl;
R¹⁵ is C₁-C₆ alkyl; and
R¹⁶ is H or C₁-C₁₀ alkyl.

2. A compound according to claim 1 wherein X² is =NOR¹, R¹⁵ is methyl, R¹⁶ is ethyl, R⁶ is H or acetyl, X¹ is -NR⁴- and R² is -C(R⁷)(R⁸)C(R⁹)(R¹⁰)C(R¹¹)(R¹²)(4-10 membered heterocyclic) wherein said heterocyclic group is substituted by 4-10 membered heterocyclic and the foregoing heterocyclic moieties are optionally substituted by 1 or 2 substituents independently selected from halo and C₁-C₃ alkyl.

3. A compound according to claim 2 wherein X¹ is -NH- and R² is -C(R⁷)(R⁸)C(R⁹)(R¹⁰)C(R¹¹)(R¹²)(4-pyridin-3-yl-imidazol-1-yl).

4. A compound according to claim 3 wherein R⁷, R⁸, R⁹ and R¹⁰ are each H, R¹¹ is methyl or ethyl, and R¹² is H, methyl or ethyl.

5. A compound according to claim 3 wherein R⁷, R⁸, R¹¹ and R¹² are each H, R⁹ is methyl or ethyl, and R¹⁰ is H, methyl or ethyl.

6. A compound according to claim 3 wherein R⁹, R¹⁰, R¹¹ and R¹² are each H, R⁷ is methyl or ethyl, and R⁸ is H, methyl or ethyl.

7. A compound according to claim 1 wherein said compound is selected from:
11-Deoxy-5-O-desosaminyl-11-(3,3-dimethyl-3-(4-pyridin-3-yl-imidazol-1-yl-propyl))hydrazo-6-O-methyl-3-oxoerythronolide A, 11,12-carbamate, 9-E-(O-methyl)oxime;
11-Deoxy-5-O-desosaminy-11-(3(R)-methyl-3-(4-pyridin-3-yl-imidazol-1-yl-propyl))hydrazo-6-O-methyl-3-oxoerythronolide A, 11,12-carbamate, 9-E-(O-methyl)oxime;
11-Deoxy-5-O-desosaminyl-11-(3(R)-ethyl-3-(4-pyridin-3-yl-imidazol-1-yl-propyl))hydrazo-6-O-methyl-3-oxoerythronolide A, 11,12-carbamate, 9-E-(O-methyl)oxime;
11-Deoxy-5-O-desosaminyl-11-(3(S)-methyl-3-(4-pyridin-3-yl-imidazol-1-yl-propyl))hydrazo-6-O-methyl-3-oxoerythronolide A, 11,12-carbamate, 9-E-(O-methyl)oxime;
11-Deoxy-5-O-desosaminyl-11-(3(S)-ethyl-3-(4-pyridin-3-yl-imidazol-1-yl-propyl))hydrazo-6-O-methyl-3-oxoerythronolide A, 11,12-carbamate, 9-E-(O-methyl)oxime;
11-Deoxy-5-O-desosaminyl-11-(2-(1-(4-pyridin-3-yl-imidazol-1-yl)-cyclopropyl)-ethyl)hydrazo-6-O-methyl-3-oxoerythronolide A, 11,12-carbamate, 9-E-(O-methyl)oxime;
11-Deoxy-5-O-desosaminyl-11-(2-(1-(4-pyridin-3-yl-imidazol-1-yl)-cyclobutyl)-ethyl)hydrazo-6-O-methyl-3-oxoerythronolide A, 11,12-carbamate, 9-E-(O-methyl)oxime;
11-Deoxy-5-O-desosaminyl-11-(2,2-dimethyl-3-(4-pyridin-3-yl-imidazol-1-yl-propyl))hydrazo-6-O-methyl-3-oxoerythronolide A, 11,12-carbamate, 9-E-(O-methyl)oxime;
11-Deoxy-5-O-desosaminyl-11-(2,2-diethyl-3-(4-pyridin-3-yl-imidazol-1-yl-propyl))hydrazo-6-O-methyl-3-oxoerythronolide A, 11,12-carbamate, 9-E-(O-methyl)oxime;
11-Deoxy-5-O-desosaminyl-11-(2,2-difluro-3-(4-pyridin-3-yl-imidazol-1-yl-propyl))hydrazo-6-O-methyl-3-oxoerythronolide A, 11,12-carbamate, 9-E-(O-methyl)oxime;
11-Deoxy-5-O-desosaminyl-11-(2,2-dichloro-3-(4-pyridin-3-yl-imidazol-1-yl-propyl))hydrazo-6-O-methyl-3-oxoerythronolide A, 11,12-carbamate, 9-E-(O-methyl)oxime;
11-Deoxy-5-O-desosaminyl-11-(2,2-dibromo-3-(4-pyridin-3-yl-imidazol-1-yl-propyl))hydrazo-6-O-methyl-3-oxoerythronolide A, 11,12-carbamate, 9-E-(O-methyl)oxime;
11-Deoxy-5-O-desosam inyl-11-(1-(4-pyridin-3-yl-imidazol-1-yl-methyl)-cyclopropyl)-methyl)hydrazo-6-O-methyl-3-oxoerythronolide A, 11,12-carbamate, 9-E-(O-methyl)oxime;
11-Deoxy-5-O-desosaminyl-11-(1-(4-pyridin-3-yl-imidazol-1-yl-methyl)-cyclobutyl)-methyl)hydrazo-6-O-methyl-3-oxoerythronolide A, 11,12-carbamate, 9-E-(O-methyl)oxime;
11-Deoxy-5-O-desosaminyl-11-(2(R)-methyl-3-(4-pyridin-3-yl-imidazol-1-yl-propyl))hydrazo-6-O-methyl-3-oxoerythronolide A, 11,12-carbamate, 9-E-(O-methyl)oxime;
11-Deoxy-5-O-desosaminyl-11-(2(R)-ethyl-3-(4-pyridin-3-yl-imidazol-1-yl-propyl))hydrazo-6-O-methyl-3-oxoerythronolide A, 11,12-carbamate, 9-E-(O-methyl)oxime;
11-Deoxy-5-O-desosaminyl-11-(2(S)-methyl-3-(4-pyridin-3-yl-imidazol-1-yl-propyl))hydrazo-6-O-methyl-3-oxoerythronolide A, 11,12-carbamate, 9-E-(O-methyl)oxime;
11-Deoxy-5-O-desosaminyl-11-(2(S)-ethyl-3-(4-pyridin-3-yl-imidazol-1-yl-propyl))hydrazo-6-O-methyl-3-oxoerythronolide A, 11,12-carbamate, 9-E-(O-methyl)oxime;
11-Deoxy-5-O-desosaminyl-11-(2(R)-fluro-3-(4-pyridin-3-yl-imidazol-1-yl-propyl))hydrazo-6-O-methyl-3-oxoerythronolide A, 11,12-carbamate, 9-E-(O-methyl)oxime;
11-Deoxy-5-O-desosaminyl-11-(2(S)-flurol-3-(4-pyridin-3-yl-imidazol-1-yl-propyl))hydrazo-6-O-methyl-3-oxoerythronolide A, 11,12-carbamate, 9-E-(O-methyl)oxime;
and the pharmaceutically acceptable salts and solvates of the foregoing compounds.

8. A pharmaceutical composition which comprises a compound of claim 1 and a pharmaceutically acceptable carrier.

9. A compound of claim 1 for use in medical treatment of a mammal, fish, or bird.

10. Use of a compound of claim 1 in the manufacture of a medicament for the treatment of a disorder selected from a bacterial infection, a protozoal infection, and a disorder related to a bacterial infection or protozoal infection in a mammal, fish, or bird.

## Patentansprüche

1. Verbindung der Formel 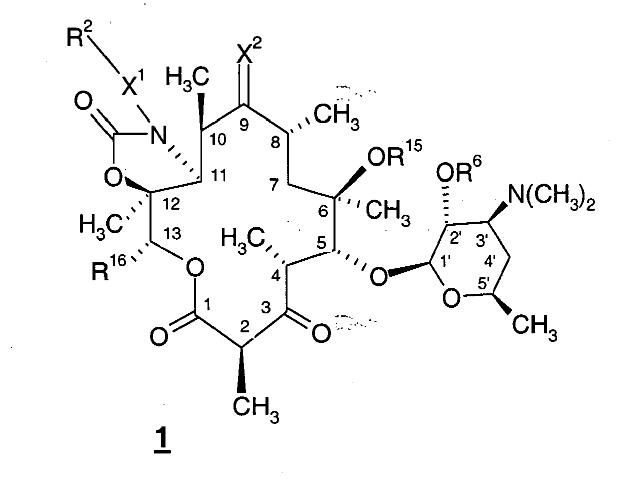 oder deren pharmazeutisch akzeptables Salz oder Solvat, worin
X¹ für -NR⁴- steht;
X² für =NOR¹ steht;
R¹ für Wasserstoff, Methyl oder Ethyl steht;
R² für die Gruppe -C(R⁷)(R⁸)C(R⁹)(R¹⁰)C(R¹¹)(R¹²)(4-10-gliedriger Heterocyclus) oder -(CR⁷R⁸)ᵣ(C₃-C₇-Cycloalkylen)(CR⁹R¹⁰)_{q}(4-10-gliedriger Heterocyclus) steht, worin q und r jeweils unabhängig ganze Zahlen von 0 bis 2 bedeuten und der heterocyclische Teil der genannten Gruppen durch einen Rest -(CR⁴R⁵)ₜ(4-10-gliedriger Heterocyclus) oder -(CR⁴R⁵)ₜ(C₆-C₁₀)-Aryl substituiert ist, worin t eine ganze Zahl von 0 bis 5 bedeutet und der Arylteil bzw. der heterocyclische Teil der genannten Gruppen jeweils optional durch 1 - 4 R³-Gruppen substituiert ist;
R³ jeweils unabhängig ausgewählt ist aus Halogen, Cyano, Nitro, Trifluormethyl, Trifluormethoxy, Azido, -C(O)R⁵, -C(O)OR⁵, -OC(O)R⁵, -NR⁵C(O)R⁴,
R⁴ -C(O)NR⁴R⁵, -NR⁴R⁵, Hydroxy, C₁-C₈-Alkyl, C₂-C₆-Alkenyl, C₂-C₆-Alkinyl, C₆-C₁₀-Aryl, 4-10-gliedrigem Heterocyclus und C₁-C₈-Alkoxy; und R⁵ jeweils unabhängig ausgewählt sind aus H und C₁-C₆-Alkyl;
R⁶ für Wasserstoff oder -C(O)(C₁-C₆-Alkyl) steht;
R⁷, R⁸, R⁹, R¹⁰, R¹¹, R¹², R¹³ und R¹⁴ jeweils unabhängig ausgewählt sind aus Wasserstoff, Halogen, und C₁-C₆-Alkyl, unter der Bedingung, dass, wenn X¹ für -NR⁴- und R² für die Gruppe -C(R⁷)(R⁸)C(R⁹)(R¹⁰)C(R¹¹)C(R¹²)(4-10-gliedriger Heterocyclus) steht, wenigstens einer der Reste R⁷, R⁸, R⁹, R¹⁰, R¹¹ und R¹² für Halogen oder C₁-C₅-Alkyl steht;
oder R⁷ und R⁸ gemeinsam mit dem Kohlenstoffatom, an das sie jeweils gebunden sind, eine C₃-C₇-Cycloalkylgruppe bilden und R⁹, R¹⁰, R¹¹, R¹², R¹³ und R¹⁴ jeweils unabhängig ausgewählt sind aus Wasserstoff, Halogen, und C₁-C₆-Alkyl;
oder R⁹ und R¹⁰ gemeinsam mit dem Kohlenstoffatom, an das sie jeweils gebunden sind, eine C₃-C₆-Cycloalkylgruppe bilden und R⁷, R⁸, R¹¹, R¹², R¹³ und R¹⁴ jeweils unabhängig ausgewählt sind aus Wasserstoff, Halogen, und C₁-C₆-Alkyl;
oder R¹¹ und R¹² gemeinsam mit dem Kohlenstoffatom, an das sie jeweils gebunden sind, eine C₃-C₇-Cycloalkylgruppe bilden und R⁷, R⁸, R⁹, R¹⁰, R¹³ und R¹⁴ jeweils unabhängig ausgewählt sind aus Wasserstoff, Halogen, und C₁-C₆-Alkyl;
R¹⁵ für C₁-C₆-Alkyl steht; und
R¹⁶ für Wasserstoff oder C₁-C₁₀-Alkyl steht.

2. Verbindung nach Anspruch 1, worin X² für =NOR¹ steht, R¹⁵ für Methyl steht, R¹⁶ für Ethyl steht, R⁶ für Wasserstoff oder Acetyl steht, X¹ für -NR⁴- steht und R² für die Gruppe -C(R⁷)(R⁸)C(R⁹)(R¹⁰)C(R¹¹)(R¹²)(4-10-gliedriger Heterocyclus) steht, worin der heterocyclische Teil durch einen 4-10-gliedrigen Heterocyclus substituiert ist und die genannten heterocyclischen Gruppen optional ein- oder zweifach unabhängig durch Halogen oder C₁-C₃-Alkyl substituiert sind.

3. Verbindung nach Anspruch 2, worin X¹ für -NH- steht und R² für -C(R⁷)(R⁸)C(R⁹)(R¹⁰)C(R¹¹)(R¹²)(4-pyridin-3-yl-imidazol-1-yl).

4. Verbindung nach Anspruch 3, worin R⁷, R⁸, R⁹ und R¹⁰ jeweils Wasserstoff bedeuten, R¹¹ für Methyl oder Ethyl und R¹² für Wasserstoff, Methyl oder Ethyl steht.

5. Verbindung nach Anspruch 3, worin R⁷, R⁸, R¹¹ und R¹² jeweils Wasserstoff bedeuten, R⁹ für Methyl oder Ethyl und R¹⁰ für Wasserstoff, Methyl oder Ethyl steht.

6. Verbindung nach Anspruch 3, worin R⁹,R¹⁰, R¹¹ und R¹² jeweils Wasserstoff bedeuten, R⁷ für Methyl oder Ethyl und R⁸ für Wasserstoff, Methyl oder Ethyl steht.

7. Verbindung nach Anspruch 1, ausgewählt aus:
11-Deoxy-5-O-desosaminyl-11-(3,3-dimethyl-3-(4-pyridin-3-yl-imidazol-1-yl-propyl))hydrazo-6-O-methyl-3-oxoerythronolid A-11,12-carbamat-9-E-(O-methyl)oxim;
11-Deoxy-5-O-desosaminyl-11-(3(R)-methyl-3-(4-pyridin-3-yl-imidazol-1-yl-propyl))hydrazo-6-O-methyl-3-oxoerythronolid A-11,12-carbamat-9-E-(O-methyl)oxim;
11-Deoxy-5-O-desosaminyl-11-(3(R)-ethyl-3-(4-pyridin-3-yl-imidazol-1-yl-propyl))hydrazo-6-O-methyl-3-oxoerythronolid A-11,12-carbamat-9-E-(O-methyl)oxim;
11-Deoxy-5-O-desosaminyl-11-(3(S)-methyl-3-(4-pyridin-3-yl-imidazol-1-yl-propyl))hydrazo-6-O-methyl-3-oxoerythronolid A-11,12-carbamat-9-E-(O-methyl)oxim;
11-Deoxy-5-O-desosaminyl-11-(3(S)-ethyl-3-(4-pyridin-3-yl-imidazol-1-yl-propyl))hydrazo-6-O-methyl-3-oxoerythronolid A-11,12-carbamat-9-E-(O-methyl)oxim;
11-Deoxy-5-O-desosaminyl-11-(2-(1-(4-pyridin-3-yl-imidazol-1-yl)-cyclopropyl)-ethyl)hydrazo-6-O-methyl-3-oxoerythronolid A-11,12-carbamat-9-E-(O-methyl)oxim;
11-Deoxy-5-O-desosaminyl-11-(2-(1-(4-pyridin-3-yl-imidazol-1-yl)-cyclobutyl)-ethyl)hydrazo-6-O-methyl-3-oxoerythronolid A-11,12-carbamat-9-E-(O-methyl)oxim;
11-Deoxy-5-O-desosaminyl-11-(2,2-dimethyl-3-(4-pyridin-3-yl-imidazol-1-yl-propyl))hydrazo-6-O-methyl-3-oxöerythronolid A-11,12-carbamat-9-E-(O-methyl)oxim;
11-Deoxy-5-O-desosaminyl-11-(2,2-diethyl-3-(4-pyridin-3-yl-imidazol-1-yl-propyl))hydrazo-6-O-methyl-3-oxoerythronolid A-11,12-carbamat-9-E-(O-methyl)oxim;
11-Deoxy-5-O-desosaminyl-11-(2,2-difluor-3-(4-pyridin-3-yl-imidazol-1-yl-propyl))hydrazo-6-O-methyl-3-oxoerythronolid A-11,12-carbamat-9-E-(O-methyl)oxim;
11-Deoxy-5-O-desosaminyl-11-(2,2-dichlor-3-(4-pyridin-3-yl-imidazol-1-yl-propyl))hydrazo-6-O-methyl-3-oxoerythronolid A-11,12-carbamat-9-E-(O-methyl)oxim;
11-Deoxy-5-O-desosaminyl-11-(2,2-dibrom-3-(4-pyhdin-3-yl-imidazol-1-yl-propyl))hydrazo-6-O-methyl-3-oxoerythronolid A-11,12-carbamat-9-E-(O-methyl)oxim;
11-Deoxy-5-O-desosaminyl-11-(1-(4-pyridin-3-yl-imidazol-1-yl-methyl)-cyclopropyl)-methyl)hydrazo-6-O-methyl-3-oxoerythronolid A-11,12-carbamat-9-E-(O-methyl)oxim;
11-Deoxy-5-O-desosaminyl-11-(1-(4-pyridin-3-yl-imidazol-1-yl-methyl)-cyclobutyl)-methyl)hydrazo-6-O-methyl-3-oxoerythronolid A-11,12-carbamat-9-E-(O-methyl)oxim;
11-Deoxy-5-O-desosaminyl-11-(2(R)-methyl-3-(4-pyridin-3-yl-imidazol-1-yl-propyl))hydrazo-6-O-methyl-3-oxoerythronolid A-11,12-carbamat-9-E-(O-methyl)oxim;
11-Deoxy-5-O-desosaminyl-11-(2(R)-ethyl-3-(4-pyridin-3-yl-imidazol-1-yl-propyl))hydrazo-6-O-methyl-3-oxoerythronolid A-11,12-carbamat-9-E-(O-methyl)oxim;
11-Deoxy-5-O-desosaminyl-11-(2(S)-methyl-3-(4-pyridin-3-yl-imidazol-1-yl-propyl))hydrazo-6-O-methyl-3-oxoerythronolid A-11,12-carbamat-9-E-(O-methyl)oxim;
11 -Deoxy-5-O-desosaminyl-11-(2(S)-ethyl-3-(4-pyridin-3-yl-imidazol-1-yl-propyl))hydrazo-6-O-methyl-3-oxoerythronolid A-11,12-carbamat-9-E-(O-methyl)oxim;
11-Deoxy-5-O-desosaminyl-11-(2(R)-fluor-3-(4-pyridin-3-yl-imidazol-1-yl-propyl))hydrazo-6-O-methyl-3-oxoerythronolid A-11,12-carbamat-9-E-(O-methyl)oxim;
11-Deoxy-5-O-desosaminyl-11-(2(S)-fluor-3-(4-pyridin-3-yl-imidazol-1-yl-propyl))hydrazo-6-O-methyl-3-oxoerythronolid A-11,12-carbamat-9-E-(O-methyl)oxim;
und den pharmazeutisch akzeptablen Salzen und Solvaten dieser Verbindungen.

8. Pharmazeutische Komposition, enthaltend eine Verbindung gemäß Anspruch 1 und einen pharmazeutisch akzeptablen Träger.

9. Verbindung nach Anspruch 1 zur Verwendung zur medikamentösen Behandlung von Säugetieren, Fischen oder Vögeln.

10. Verwendung einer Verbindung nach Anspruch 1 zur Herstellung eines Arzneimittels zur Behandlung einer Störung, die ausgewählt ist aus bakteriellen Infektionen, Protozoeninfektionen und mit bakteriellen Infektionen und Protozoeninfektionen in Zusammenhang stehenden Störungen bei Säugetieren, Fischen oder Vögeln.

## Revendications

1. Composé de formule 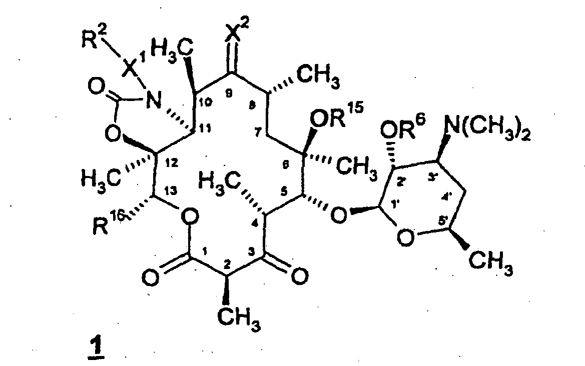 ou sel ou solvat pharmaceutiquement acceptable d'un tel composé, dans lequel :
X¹ représente -NR⁴- ;
X² représente =NOR¹;
R¹ représente H, méthyle ou éthyle ;
R² représente -C (R⁷) (R⁸)C(R⁹) (R¹⁰)C(R¹¹) (R¹²) (hétérocyclique ayant de 4. à 10 éléments) ou -(CR⁷R⁸)ᵣ(cycloalkylène en C₃₋C₇) (CR⁹R¹⁰)_{q}(hétérocyclique ayant de 4 à 10 éléments), où q et r sont chacun des nombres entiers compris indépendamment entre 0 et 2, et le motif hétérocyclique, des groupes précités est substitué par -(CR⁴R⁵)ₜ (hétérocyclique ayant de 4 à 10 éléments) ou (CR⁴R⁵)ₜ(aryle en C₆-C₁₀) où t est un nombre entier allant de 0 à 5, et les motifs aryle et hétérocyclique de chacun des groupes précités sont facultativement substitués par de 1 à 4 groupes R³;
chaque R³ est indépendamment sélectionné dans le groupe consistant en halogéno, cyano, nitro, trifluorométhyle, trifluorométhoxy, azido, -C(O)R⁵, -C(O)OR⁵, -OC(O)R⁵, -NR⁵C(O)R⁴, -C(O)NR⁴R⁵, NR⁴R⁵, hydroxy, alkyle en C₁-C₆, alkényle en C₂-C₆, alkynyle en C₂-C₆, aryle en C₆-C₁₀, hétérocyclique ayant de 4 à 10 éléments et alcoxy en C₁-C₆ ;
chaque R⁴ et R⁵ est indépendamment sélectionné parmi H et alkyle en C₁-C₆ ;
R⁶ représente H ou -C(O) (alkyle en C₁-C₆) ;
R⁷, R⁸ , R⁹, R¹⁰, R¹¹, R¹², R¹³ et R¹⁴ sont chacun indépendamment sélectionnés parmi H, halogéno et alkyle en C₁-C₆, étant entendu que (a) lorsque X¹ représente -NR⁴- et que R² représente -C(R⁷) (R⁸)C (R⁹) (R¹⁰) C (R¹¹) (R¹²) (hétérocyclique ayant de 4 à 10 éléments), l'un au moins de R⁷, R⁸, R⁹, R¹⁰, R¹¹ et R¹² représente halogéno ou alkyle en C₁-C₆ ;
ou R⁷ et R⁸ sont pris ensemble avec le carbone auquel ils sont fixés pour former un groupe cycloalkyle en C₃-C₇ et R⁹, R¹⁰, R¹¹, R¹², R¹³ et R¹⁴ sont chacun indépendamment sélectionnés parmi H, halogéno et alkyle en C₁-C₆ ;
ou R⁹ et R¹⁰ sont pris ensemble avec le carbone auquel ils sont fixés pour former un groupe cycloalkyle en C₃-C₆ et R⁷, R⁸, R¹¹, R¹², R¹³ et R¹⁴ sont chacun indépendamment sélectionnés parmi H, halogéno et alkyle en C₁-C₆ ;
ou R¹¹ et R¹² sont pris ensemble avec le carbone auquel ils sont fixés pour former un groupe cycloalkyle en C₃-C₇ et R⁷ R⁸ R⁹, R¹⁰, R¹³ et R¹⁴ sont chacun indépendamment sélectionnés parmi H, halogéno et alkyle en C₁-C₆ ;
R¹⁵ représente alkyle en C₁-C₆ ; et
R¹⁶ représente H ou alkyle en C₁-C₁₀.

2. Composé selon la revendication 1, dans lequel X² représente =NOR¹, R¹⁵ représente méthyle, R¹⁶ représente éthyle, R⁶ représente H ou acétyle, X¹ représente -NR⁴- et R² représente -C(R⁷) (R⁸)C(R⁹) (R¹⁰)C(R¹¹) (R¹²) (hétérocyclique ayant de 4 à 10 éléments) où ledit groupe hétérocyclique est substitué par un groupe hétérocyclique ayant de 4 à 10 éléments et les motifs hétérocycliques précités sont facultativement substitués par 1 ou 2 substituants indépendamment sélectionnés parmi halogéno et alkyle en C₁-C₃.

3. Composé selon la revendication 2, dans lequel X¹ représente -NH- et R² représente -C(R⁷) (R⁸)C(R⁹) (R¹⁰)C(R¹¹) (R¹²) (4-pyridin-3-yl-imidazol-1-yle).

4. Composé selon la revendication 3, dans lequel R⁷, R⁸, R⁹ et R¹⁰ représentent chacun H, R¹¹ représente méthyle ou éthyle, et R¹² représente H, méthyle ou éthyle.

5. Composé selon la revendication 3, dans lequel R⁷, R⁸, R¹¹ et R¹² représentent chacun H, R⁹ représente méthyle ou éthyle, et R¹⁰ représente H, méthyle ou éthyle.

6. Composé selon la revendication 3, dans lequel R⁹, R¹⁰, R¹¹ et R¹² représentent chacun H, R⁷ représente méthyle ou éthyle, et R⁸ représente H, méthyle ou éthyle.

7. Composé selon la revendication 1, qui est sélectionné parmi :
• 11-déoxy-5-O-désosaminyl-11-[3,3-diméthyl-3-(4-pyridin-3-yl-imidazol-1-yl-propyl)]hydrazo-6-O-méthyl-3-oxoérythronolide A, 11,12-carbamate, 9-E-(O-méthyl)oxime ;
• 11-déoxy-5-O-désosaminyl-11-[3(R)-méthyl-3-(4-pyridin-3-yl-imidazol-1-yl-propyl)]hydrazo-6-O-méthyl-3-oxoérythronolide A, 11,12-carbamate, 9-E-(O-méthyl)oxime ;
• 11-déoxy-5-0-désosaminyl-11-[3(R)-éthyl-3-(4-pyridin-3-yl-imidazol-1-yl-propyl)]hydrazo-6-O-méthyl-3-oxoérythronolide A, 11,12-carbamate, 9-E-(O-méthyl)oxime ;
• 11-déoxy-5-0-désosaminyl-11-[3(S)-méthyl-3-(4-pyridin-3-yl-imidazol-1-yl-propyl)]hydrazo-6-O-méthyl-3-oxoérythronolide A, 11,12-carbamate, 9-E-(O-méthyl)oxime ;
• 11-déoxy-5-O-désosaminyl-11-[3(S)-éthyl-3-(4-pyridin-3-yl-imidazol-1-yl-propyl)]hydrazo-6-O-méthyl-3-oxoérythronolide A, 11,12-carbamate, 9-E-(O-méthyl)oxime ;
• 11-déoxy-5-O-désosaminyl-11-{2-[1-(4-pyridin-3-yl-imidazol-1-yl)-cyclopropyl]-éthyl}hydrazo-6-O-méthyl-3-oxoérythronolide A, 11,12-carbamate, 9-E-(O-méthyl)oxime ;
• 11-déoxy-5-O-désosaminyl-11-{2-[1-(4-pyridin-3-yl-imidazol-1-yl)-cyclobutyl]-éthyl}hydrazo-6-O-méthyl-3-oxoérythronolide A, 11,12-carbamate, 9-E-(O-méthyl)oxime ;
• 11-déoxy-5-O-désosaminyl-11-[2,2-diméthyl-3-(4-pyridin-3-yl-imidazol-1-yl-propyl)]hydrazo-6-O-méthyl-3-oxoérythronolide A, 11,12-carbamate, 9-E-(O-méthyl)oxime ;
• 11-déoxy-5-O-désosaminyl-11-[2,2-diéthyl-3-(4-pyridin-3-yl-imidazol-1-yl-propyl)]hydrazo-6-O-méthyl-3-oxoérythronolide A, 11,12-carbamate, 9-E-(O-méthyl)oxime ;
• 11-déoxy-5-O-désosaminyl-11-[2,2-difluoro-3-(4-pyridin-3-yl-imidazol-1-yl-propyl)]hydrazo-6-O-méthyl-3-oxoérythronolide A, 11,12-carbamate, 9-E-(O-méthyl)oxime ;
• 11-déoxy-5-O-désosaminyl-11-[2,2-dichloro-3-(4-pyridin-3-yl-imidazol-1-yl-propyl)]hydrazo-6-O-méthyl-3-oxoérythronolide A, 11,12-carbamate, 9-E-(O-méthyl)oxime ;
• 11-déoxy-5-O-désosaminyl-11-[2,2-dibromo-3-(4-pyridin-3-yl-imidazol-1-yl-propyl)]hydrazo-6-O-méthyl-3-oxoérythronolide A, 11,12-carbamate, 9-E-(O-méthyl)oxime ;
• 11-déoxy-5-O-désosaminyl-11-{[1-(4-pyridin-3-yl-imidazol-1-yl-méthyl)-cyclopropyl]-méthyl}hydrazo-6-O-méthyl-3-oxoérythronolide A, 11,12-carbamate, 9-E-(O-méthyl)oxime ;
• 11-déoxy-5-O-désosaminyl-11-{[1-(4-pyridin-3-yl-imidazol-1-yl-méthyl)-cyclobutyl]-méthyl}hydrazo-6-O-méthyl-3-oxoérythronolide A, 11,12-carbamate, 9-E-(O-méthyl)oxime ;
• 11-déoxy-5-O-désosaminyl-11-[2(R)-méthyl-3-(4-pyridin-3-yl-imidazol-1-yl-propyl)]hydrazo-6-O-méthyl-3-oxoérythronolide A, 11,12-carbamate, 9-E-(O-méthyl)oxime ;
• 11-déoxy-5-O-désosaminyl-11-[2(R)-éthyl-3-(4-pyridin-3-yl-imidazol-1-yl-propyl)]hydrazo-6-O-méthyl-3-oxoérythronolide A, 11,12-carbamate, 9-E-(O-méthyl)oxime ;
• 11-déoxy-5-O-désosaminyl-11-[2(S)-méthyl-3-(4-pyridin-3-yl-imidazol-1-yl-propyl)]hydrazo-6-0-méthyl-3-oxoérythronolide A, 11,12-carbamate, 9-E-(O-méthyl)oxime ;
• 11-déoxy-5-O-désosaminyl-11-[2(S)-éthyl-3-(4-pyridin-3-yl-imidazol-1-yl-propyl)]hydrazo-6-O-méthyl-3-oxoérythronolide A, 11,12-carbamate, 9-E-(O-méthyl)oxime ;
• 11-déoxy-5-O-désosaminyl-11-[2(R)-fluoro-3-(4-pyridin-3-yl-imidazol-1-yl-propyl)]hydrazo-6-O-méthyl-3-oxoérythronolide A, 11,12-carbamate, 9-É-(O-méthyl)oxime ;
• 11-déoxy-5-O-désosaminyl-11-[2(S)-fluoro-3-(4-pyridin-3-yl-imidazol-l-yl-propyl)]hydrazo-6-0-méthyl-3-oxoérythronolide A, 11,12-carbamate, 9-E-(O-méthyl)oxime ;
et les sels et solvats pharmaceutiquement acceptables des composés précités.

8. Composition pharmaceutique qui comprend un composé selon la revendication 1 et un support pharmaceutiquement acceptable.

9. Composé selon la revendication 1, pour utilisation dans le traitement médical d'un mammifère, d'un poisson ou d'un oiseau.

10. Utilisation d'un composé selon la revendication 1, dans la fabrication d'un médicament pour le traitement d'un trouble sélectionné entre une infection bactérienne, une infection à protozoaires ou un trouble associé à une infection bactérienne ou à une infection à protozoaires chez un mammifère, un poisson ou un oiseau.
